(19) **Europäisches Patentamt / European Patent Office / Office européen des brevets**

(11) **EP 3 467 071 B1**

(12) **EUROPEAN PATENT SPECIFICATION**

(45) Date of publication and mention
of the grant of the patent:
**04.06.2025 Bulletin 2025/23**

(21) Application number: **17806177.6**

(22) Date of filing: **04.04.2017**

(51) International Patent Classification (IPC):
*C09K 3/00* (2006.01)    *C01G 9/02* (2006.01)
*C09C 1/02* (2006.01)    *C09C 1/04* (2006.01)
*C09C 1/24* (2006.01)    *C09C 1/40* (2006.01)
*C09C 3/06* (2006.01)    *C01G 23/053* (2006.01)
*C01G 49/04* (2006.01)    *A61K 8/19* (2006.01)
*A61K 8/27* (2006.01)    *A61K 8/29* (2006.01)
*A61Q 1/02* (2006.01)    *A61Q 17/04* (2006.01)
*C09D 5/32* (2006.01)    *C09C 1/36* (2006.01)

(52) Cooperative Patent Classification (CPC):
C01G 9/02; A61K 8/19; A61K 8/29; A61Q 17/04;
C01G 23/0532; C01G 49/04; C09C 1/02;
C09C 1/04; C09C 1/043; C09C 1/24; C09C 1/3607;
C09C 1/3653; C09C 1/3661; C09C 1/3692;
C09C 1/40;        (Cont.)

(86) International application number:
**PCT/JP2017/014157**

(87) International publication number:
**WO 2017/208616 (07.12.2017 Gazette 2017/49)**

(54) **COLORANT / UV PROTECTANT**

FARBSTOFF-/UV-SCHUTZMITTEL

AGENT COLORANT/PROTECTEUR ANTI-UV

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.06.2016 JP 2016111346
03.06.2016 PCT/JP2016/066542
29.11.2016 JP 2016231897**

(43) Date of publication of application:
**10.04.2019 Bulletin 2019/15**

(73) Proprietor: **M. Technique Co., Ltd.
Izumi-shi, Osaka 594-1144 (JP)**

(72) Inventors:
 • **ENOMURA Masakazu**
  **Izumi-shi
  Osaka 594-1144 (JP)**

 • **HONDA Daisuke**
  **Izumi-shi
  Osaka 594-1144 (JP)**
 • **ARAKI Kaeko**
  **Izumi-shi
  Osaka 594-1144 (JP)**

(74) Representative: **Marks & Clerk LLP
15 Fetter Lane
London EC4A 1BW (GB)**

(56) References cited:
| | |
|---|---|
| WO-A1-03/022954 | WO-A1-03/083008 |
| WO-A1-2004/103907 | JP-A- 2005 263 612 |
| JP-A- 2005 281 435 | JP-A- 2010 100 467 |
| JP-A- 2011 068 628 | JP-A- 2012 250 860 |
| JP-A- 2017 043 505 | JP-A- H09 188 517 |
| US-A1- 2019 144 691 | US-B1- 6 534 044 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

(52) Cooperative Patent Classification (CPC): (Cont.)
    **C09C 3/06; C09C 3/063;** A61K 2800/413;
    A61K 2800/591; A61Q 1/02; C01P 2002/52;
    C01P 2002/54; C01P 2002/72; C01P 2002/85;
    C01P 2004/04; C01P 2006/62; C01P 2006/63;
    C01P 2006/64; C01P 2006/65; C01P 2006/66

**Description**

TECHNICAL FIELD

The present invention relates to a coloring ultraviolet protective agent.

BACKGROUND ART

Ultraviolet absorption characteristics and color characteristics such as hue of oxide particles change, by selecting a type of a metal element contained in the oxide particles. Therefore, by utilizing such characteristics, the oxide particles are used in broad fields as an outer wall of a building material, a signboard, a paint and film used for a vehicle, glass and the like, or a sunscreen agent, lipstick and foundation in the field of cosmetics, and the like. In recent years, when used for a glass for use in a building such as an office building and house, a vehicle such as an automobile, a coating body for a building and vehicle, a paint, clear coating film for use in an exterior wall, signboard, equipment and the like, or the like, a demand for vividness of colors and excellent designability is increasing in addition to the above mentioned ultraviolet absorption ability. Even in the case of aiming application to a human body like a cosmetic, etc., a demand for aesthetic appearance, texture and safety is increasing in addition to the above mentioned ultraviolet absorption ability.

A method of micronizing an oxide such as zinc oxide and iron oxide for imparting various properties to the oxide (see Patent Literatures 1 and 2), and solid solution oxide particles in which a plurality of different metal elements are solid solved in the oxide (see Patent Literatures 3, 4 and 5) have been proposed.

By the way, in general, when ultraviolet absorption ability per unit mass in the wavelength range of 200 nm to 380 nm is higher, namely, a "molar absorption coefficient" is higher, the more ultraviolet rays can be absorbed by even a small amount. Therefore, when a molar absorption coefficient is high, since even small amount exhibits ultraviolet absorption ability similar to or more than that of the conventional one, a haze value can be small, and transparency of a transparent material such as a coating material such as a coating film, a transparent resin, film or glass can be enhanced, and coloring for enhancing aesthetic appearance and designability will become enabled.

However, Patent Literature 1 and Patent Literature 2 describe characteristics in relation to reflectivity and color difference of the powder of oxide particles and silica coated oxide particles. Patent Literature 3 and Patent Literature 4 describe color characteristics indicated by the L*a*b* color system and reflectivity for near infrared lights in the wavelength range of 780 nm to 2,500 nm regarding solid solution oxide particles formed by solid solving various metal elements. However, any characteristics as a dispersion of oxide particles are not described in these patent literatures at all. Even if transparency of microparticle dispersion can be improved by micronization, it is difficult to completely absorb or shield ultraviolet rays of 380 nm or less, because of its low ultraviolet absorption ability. For its absorption or shield, a large amount of ultramicroparticles per unit area must be used, or a film thickness becomes too thick, or an amount of usage increases. Therefore, there are problems such as lack of practicality based on transparency issue. Further, Patent Literature 5 describes that cobalt solid solution zinc oxide particles can be obtained by pulverizing cobalt solid solution zinc oxide obtained by heat treatment at 800 °C as described in the examples. Since the coarse solid solution oxide is pulverized by the heat treatment at a very high temperature to obtain the particles, different elements are not uniformly distributed in each particle. Therefore, Patent Literature 5 does not aim at controlling strict color characteristics, and it is difficult to improve a molar absorption coefficient in the wavelength range of 200 nm to 380 nm of the solid solution oxide particles obtained by pulverization when the particles are dispersed in a dispersion medium.

Furthermore, in a conventional method such as a batch method as a method of producing a solid solution oxide composed of a plurality of different elements, it is extremely difficult to produce a solid solution oxide in which a plurality of different elements are solid solved throughout a particle, because it is difficult to precipitate oxides of different elements at the same time as a prerequisite. For this reason, it is virtually impossible to strictly control color characteristics.

Patent Literatures 6 and 7 filed by the present applicant discloses a method of producing uniform oxide nanoparticles using a method of precipitating various nanoparticles such as oxide between two processing surfaces being capable of approaching to and separating from each other and rotating relative to each other. However, Patent Literature 6 describes separate production of an oxide and hydroxide. Patent Literature 7 describes production of uniform oxide. However, a method of producing an oxide in relation with controlling color characteristics is not described in these patent literatures.

Further, the ultraviolet absorbing ability of oxide particles necessary for using as a composition for ultraviolet shielding should be actually evaluated by an average molar absorption coefficient in the wavelength range of 200 nm to 380 nm. However, since in these conventional technique, the ability is evaluated by transmittance for lights in the ultraviolet region, or evaluated by using a single light, it is difficult to appropriately design a proper amount and blending of oxide particles necessary for obtaining a composition such as a desired ultraviolet protective composition.

Patent Literature 8 provides metal oxide particles in which a component originating in metal elements (M') except a metal element (M) is contained in the particle of the oxide of the metal element (M). The metal element (M) is at least one kind selected from the group comprising Zn, Ti, Ce, In, Sn, Al and Si, and the metal elements (M') are different from the metal element (M) and at least two kinds selected from the group comprising Co, Cu, Fe, Bi, In, Al, Ga, Ti, Sn, Ag, Mn, Ni and Ce. Patent Literature 9 provides a cosmetic material comprising silica-coated metal oxide particles further surface-treated with a hydrophobicizing agent, and metal oxide particles having a specified infrared absorption spectrum intensity ratio and refractive index which are further treated with a hydrophobicizing agent, and a process for their production. Specifically it provides a silica-coated metal oxide sol which gives such particles, and a process for its production.

CITATION LIST

PATENT LITERATURE

Patent Literature 1: JP 2009-263547
Patent Literature 2: WO 1998/26011
Patent Literature 3: JP 2013-249393
Patent Literature 4: JP 2013-520532
Patent Literature 5: JP 2001-58821
Patent Literature 6: JP 4868558
Patent Literature 7: WO 2009/008393
Patent Literature 8: JP2005263612A
Patent Literature 9: US6534044B1

SUMMARY OF THE INVENTION

TECHNICAL PROBLEM

In view of such circumstances, an object of the present invention is to provide a coloring ultraviolet protective agent, comprising M2 doped oxide particles in which oxide particles (M1Ox) comprising at least M1 being a metal element or metalloid element, are doped with M2 being a metal element or metalloid element other than M1, wherein an average molar absorption coefficient in the wavelength range of 200 nm to 380 nm is increased, and wherein color characteristics in the visible region are controlled. Namely, an object of the present invention is to control an average molar absorption coefficient in the wavelength range of 200 nm to 380 nm and color characteristics in the visible region, by preparing M2 doped oxide particles for the purpose of maximally improving the original properties of the oxides and of supplementing such properties. Further, in view of such circumstances, an object of the present invention is to provide a coloring ultraviolet protective agent, comprising M2 doped oxide particles, wherein an average molar absorption coefficient in the wavelength range of 200 nm to 380 nm is increased, and wherein color characteristics in the visible region are strictly controlled. And an object of the present invention is particularly to provide M2 doped oxide particles suitable as a coloring ultraviolet protective agent composition.

SOLUTION TO THE PROBLEM

The inventors of the present invention have found that by using M2 doped oxide particles in which oxide particles (M1Ox) comprising at least M1 being a metal element or metalloid element, are doped with at least one M2 selected from metal elements or metalloid elements other than M1, an average molar absorption coefficient in the wavelength range of 200 nm to 380 nm of a dispersion in which the M2 doped oxide particles are dispersed in a dispersion medium, can be increased significantly, and color characteristics in the visible region can be strictly controlled. Thereby, the inventors have completed the present invention.

Namely, the present invention is a coloring ultraviolet protective agent, which is useful for shielding ultraviolet rays and coloring, wherein:

the coloring ultraviolet protective agent comprises M2 doped oxide particles in which oxide particles (M1Ox) comprising at least M1 being a metal element or metalloid element, are doped with at least one M2 selected from metal elements or metalloid elements other than M1,
x is an arbitrary positive number,
at least a part of the surface of the M2 doped oxide particles is coated with a compound containing an amorphous silicon oxide,
a molar ratio of M1 and M2 (M2/M1) of the M2 doped oxide particles is in the range of 0.01 or more and 1.00 or less, and

the M2 doped oxide particles are either one of the following:

(A) M1 is iron (Fe), color characteristics in the visible region of the M2 doped oxide particles are controlled in the range of $38 \leq L^* \leq 44$, $4 \leq a^* \leq 14$, or $4 \leq b^* \leq 12$ in the $L^*a^*b^*$ color system, and an average molar absorption coefficient in the wavelength range of 200 nm to 380 nm of a dispersion in which the M2 doped oxide particles are dispersed in a dispersion medium, is 1,000 L/(mol·cm) or more; or

B) M1 is titanium (Ti), color characteristics in the visible region of the M2 doped oxide particles are controlled in the range of $40 \leq L^* \leq 95$, $-35 \leq a^* \leq 35$, or $-35 \leq b^* \leq 35$ in the $L^*a^*b^*$ color system, and an average molar absorption coefficient in the wavelength range of 200 nm to 380 nm of a dispersion in which the M2 doped oxide particles are dispersed in a dispersion medium, is 3,500 L/(mol·cm) or more

With the present invention the an average molar absorption coefficient in the wavelength range of 200 nm to 380 nm of a dispersion in which the M2 doped oxide particles are dispersed in a dispersion medium is improved as compared with one of a dispersion in which the oxide particles (M1Ox) are dispersed in a dispersion medium, and a hue or chroma of color characteristics in the visible region of the M2 doped oxide particles is controlled.

In the present invention, the M2 doped oxide particles are oxide particles in which a molar ratio of M1 and M2 (M2/M1) in the M2 doped oxide particles is preferably controlled, wherein an increase rate of the average molar absorption coefficient in the wavelength range of 200 nm to 380 nm of a dispersion in which the M2 doped oxide particles are dispersed in a dispersion medium, over an average molar absorption coefficient in the same wavelength range of a dispersion in which the oxide particles (M1Ox) are dispersed in a dispersion medium is controlled.

In the present invention, a molar ratio of M1 and M2 (M2/M1) of the M2 doped oxide particles is in the range of 0.01 or more and 1.00 or less.

Further, in the present invention, an average molar absorption coefficient increase rate which is an increase rate of the average molar absorption coefficient in the wavelength range of 200 nm to 380 nm of a dispersion in which the M2 doped oxide particles are dispersed in a dispersion medium, over an average molar absorption coefficient in the same wavelength range of a dispersion in which the oxide particles (M1Ox) are dispersed in a dispersion medium is preferably 110% or more.

In the present invention, the M2 doped oxide particles are preferably solid solution oxide particles in which M1 and M2 are detected throughout the M2 doped oxide particles in STEM mapping.

In the present invention, a primary particle diameter of the M2 doped oxide particle is preferably 1 nm or more and 100 nm or less.

In the present invention, the coloring ultraviolet protective agent is silicon compound coated M2 doped oxide particles in which at least a part of the surface of the M2 doped oxide particles is coated with a silicon compound, wherein an average molar absorption coefficient in the wavelength range of 200 nm to 380 nm of a dispersion in which the silicon compound coated M2 doped oxide particles are dispersed in a dispersion medium, is increased over one of a dispersion of the M2 doped oxide particles not coated with the silicon compound. The coloring ultraviolet protective agent is more preferably one having an average molar absorption coefficient increase rate of 120% or more.

Further, the present invention can be carried out as a coloring ultraviolet protective agent composition, comprising the coloring ultraviolet protective agent.

ADVANTAGEOUS EFFECTS OF THE INVENTION

The present invention can provide M2 doped oxide particles in which oxide particles (M1Ox) comprising at least M1 being a metal element or metalloid element, are doped with at least one M2 selected from metal elements or metalloid elements other than M1, and thereby an average molar absorption coefficient in the wavelength range of 200 nm to 380 nm of the M2 doped oxide particles is increased compared with the oxide particles (M1Ox). In particular, since an average molar absorption coefficient in the ultraviolet wavelength region of 200 nm to 380 nm can be increased, composition design appropriate to diversifying application and objective properties of the M2 doped oxide particles can be facilitated, compared with conventional oxide particles. In particular, by applying the M2 doped oxide particles of the present invention to a composition for coating or for a transparent material for ultraviolet protection, a composition for coating or a transparent material can be provided, which has high transparency, does not impair texture or appearance of a raw material or designability of a product, can be used effectively for a coating material, a transparent material, etc., and can realize effective coloring. Since color characteristics can be strictly controlled in the state of a molar absorption coefficient raised to this level, design of a composition for coating or a transparent material becomes facilitated. Namely, by merely blending a very small amount of the M2 doped oxide particles, protection of ultraviolet and appropriate coloring become possible.

BRIEF DESCRIPTION OF THE DRAWINGS

FIG 1 shows an STEM photograph and mapping results of the cobalt aluminum doped zinc oxide particles obtained in Example 1-11 of the present invention.

FIG 2 shows a linear analysis result of the cobalt aluminum doped zinc oxide particles obtained in Example 1-11 of the present invention.

FIG 3 shows an XRD measurement result of the cobalt aluminum doped zinc oxide particles obtained in Example 1-11 of the present invention, and the zinc oxide particles obtained in Comparative Example 1.

FIG 4 shows a graph of molar absorption coefficients of the dispersions obtained by dispersing in propylene glycol the cobalt doped zinc oxide particles obtained in Example 1-1 to 1-5 of the present invention, and the zinc oxide particles obtained in Comparative Example 1.

FIG 5 shows a chart of an L*a*b* color system chromaticity diagram, plotting L* values, a* values, b* values of the M2 doped zinc oxide particles obtained in Examples 1-1 to 1-19 of the present invention and the zinc oxide particles obtained in Comparative Example 1.

FIG 6 shows a chart of an L*a*b* color system chromaticity diagram, plotting L* values, a* values, b* values of the M2 doped iron oxide particles obtained in Examples 2-1 to 2-11 of the present invention and the iron oxide particles obtained in Comparative Example 2.

FIG 7 shows a chart of an L*a*b* color system chromaticity diagram, plotting L* values, a* values, b* values of the M2 doped titanium oxide particles obtained in Examples 3-1 to 3-17 of the present invention and the titanium oxide particles obtained in Comparative Example 3.

DESCRIPTION OF THE INVENTION

Hereinafter, the present invention is explained by embodiments of the present invention based on the drawings as an example. However, embodiments of the present invention are not limited only to the embodiments described hereinafter.

(Coloring ultraviolet protection agent: M2 doped oxide particles)

The M2 doped oxide particles of the present invention are M2 doped oxide particles in which oxide particles (M1Ox) comprising at least M1 being a metal element or metalloid element, are doped with at least one M2 selected from metal elements or metalloid elements other than M1, wherein their average molar absorption coefficient in the wavelength range of 200 nm to 380 nm and their color characteristics in the visible region are controlled by controlling a molar ratio of M1 and M2 (M2/M1). When the M2 doped oxide particles of the present invention is used as a coloring ultraviolet protection agent, for a composition for coating such as a coating film, coating body, a composition for application on human skin, etc., or a composition for a transparent material such as a transparent resin, glass, clear coating film, etc., the composition has high shielding ability against lights in the ultraviolet wavelength region of 200 nm to 380 nm, designability, texture or appearance is not impaired, and effective coloring is possible. Thereby, a composition for coating or for a transparent material can be provided, which can be used for a material to be coated or a transparent material. In the present invention, a molar ratio of M1 and M2 (M2/M1) is in the range of 0.01 or more and 1.00 or less.

(Embodiment-1 of M2 doped oxide particles)

M1 is iron (Fe) or titanium (Ti). The metal element or metalloid element (M2) includes one or more elements selected from metal elements and metalloid elements in the chemical periodic table. Namely, in the present invention, the oxide is not limited to the oxide consisting only of the M1 and M2, and the present invention can be also carried out using an oxide containing other element(s) M3, M4, ... Mn different from M1 and M2. The numbers attached to M are merely numbers for identification. A metalloid element in the present invention is not particularly limited, but preferably includes metalloid elements such as Ge, As, Sb, Te, Se, Si and the like. In the present invention, the M1, M2, or Mn may be contained in a coating layer that coats at least a part of the surface of the M2 doped oxide particles described later.

(Embodiment-2 of M2 doped oxide particles)

The M2 doped oxide particles of the present invention may be composed only of oxides, but is not limited to particles composed only of oxides. As the M2 doped oxide particles of the present invention, oxide particles containing a compound other than oxides may be also used to the extent that the compound does not affect the present invention. For example, M2 doped oxide particles in which oxide particles (M1Ox) containing a compound other than oxides are doped with a metal element or metalloid element, may be used. Examples of the above compound other than oxides include a hydroxide, nitride, carbide, various salts such as a nitrate and sulfate, and a hydrate and organic solvate.

(Embodiment-3 of M2 doped oxide particles)

While the M2 doped oxide particles of the present invention are oxide particles in which M1Ox is doped with M2, it is preferable to form a solid solution in which M1 and M2 are uniformly distributed in one oxide particle, in terms of enhancement of an average molar absorptivity coefficient in the wavelength range of 200 nm to 380 nm and strict control of color characteristics in the visible region. The solid solution may be solid solution oxide particles in which the M1 and M2 exist in the inside of the particles, in the vicinity of the surface layer of the particles, or in a local part of the particles. A method of evaluating that the particle is uniform or a solid solution, is preferably a method that a plurality of particles are observed using a transmission electron microscope (TEM) or a scanning transmission electron microscope (STEM), and that an abundance ratio and an existing position of M1 and M2 in each particle is confirmed using an energy dispersive X-ray analyzer (EDS). Such examples include a method of evaluating uniformity by specifying an abundance ratio (molar ratio) of M1 and M2 contained in one oxide particle and calculating an average value and variation coefficient of a molar ratio of M1 and M2 in a plurality of oxide particles, and a method of specifying an existing position of M1 or M2 contained in oxide particles by mapping. In the present invention, the M2 doped oxide particles are preferably solid solution oxide particles in which M1 and M2 are detected throughout the M2 doped oxide particles in STEM mapping.

(Average molar absorption coefficient)

A molar absorption coefficient can be calculated from an absorbance and a molar concentration of a substance to be measured in a measurement sample in ultraviolet-visible absorption spectrum measurement, by Formula 1 below.

$$\varepsilon = A / (c \cdot l) \qquad \text{(Formula 1)}$$

In Formula 1, $\varepsilon$ is a constant specific to the substance, and is referred to as a molar absorption coefficient. Since it means an absorbance of a dispersion at 1 mol/L with a thickness of 1 cm, the unit is L/(mol·cm). A is an absorbance in ultraviolet-visible absorption spectrum measurement. c is a molar concentration of a sample (mol/L). l is a length through which a light is transmitted (optical path length), typically a thickness of a cell in measuring the ultraviolet-visible absorption spectrum. In the present invention, in order to show ability to absorb lights in the ultraviolet wavelength region of 200 nm to 380 nm, a simple average of the molar absorption coefficients for a plurality of wavelengths in the measurement wavelength region of 200 nm to 380 nm is calculated and evaluated as an "average molar absorption coefficient".

(Average molar absorption coefficient increase rate)

The M2 doped oxide particles of the present invention are preferably M2 doped oxide particles in which an "average molar absorption coefficient increase rate" which is an increase rate of an average molar absorption coefficient in the wavelength region of 200 nm to 380 nm of the M2 doped oxide particles, over an average molar absorption coefficient in the same wavelength region of the oxide particles (M1Ox) is controlled. An average molar absorption coefficient increase rate which is an increase rate of an average molar absorption coefficient in the wavelength range of 200 nm to 380 nm of a dispersion in which the M2 doped oxide particles are dispersed in a dispersion medium, over an average molar absorption coefficient in the same wavelength range of a dispersion of the oxide particles (M1Ox) is preferably 110% or more, or 120% or more.

(Color characteristics other than molar absorption coefficient)

In the present invention, in the same manner as a molar absorption coefficient and an average molar absorption coefficient in the ultraviolet region in the wavelength range from 200 nm to 380 nm, by controlling a molar ratio (M2/M1), color characteristics such as a reflectivity, average reflectivity, transmittance and average transmittance in a specific region in the visible region of the wavelengths of 380 nm to 780 nm, and hue H (= b*/a*) or chroma C (= ((a*)$^2$ + (b*)$^2$)$^{1/2}$) in the L*a*b* system can be accurately and strictly controlled. Thereby, particularly M2 doped oxide particles suitable for a composition for coating or a transparent material for ultraviolet protection can be provided. In the present invention, an average molar absorption coefficient in the wavelength range of 200 nm to 380 nm of the M2 doped oxide particles is controlled by controlling a molar ratio (M2/M1) of the M2 doped oxide particles, and in addition, these color characteristics are controlled, and the M2 doped oxide particles have ability to efficiently protect from lights in the ultraviolet region.

Thereby, the present invention is suitable for use as a composition for coating or a transparent material which does not impair aesthetic appearance, texture, or designability. Since positive coloring can be made depending on the purpose, the present invention is also suitable for use as a composition for coloring.

(Color characteristics: Hue or chroma)

A hue or chroma in the present invention may be indicated by a hue H (= $b*/a*$, $b*>0$, $a*>0$) or chroma C = $((a*)^2 + (b*)^2)^{1/2}$ in the L*a*b* color system. Here, the L*a*b* color system is one of the uniform color space and L* indicates a value representing brightness, and a larger numerical value indicates as being brighter. Also, a* and b* indicate chromaticity. In the present invention, the color system is not limited to the L*a*b* color system. Color characteristics may be evaluated using other color systems such as XYZ system. Further, in the present invention, by controlling color characteristics to be in the range of $40 \leq L* \leq 95$ in the L*a*b* color system, coloring can be controlled from dark color to bright color. By controlling color characteristics in the range of $-35 \leq a* \leq 35$ or $-35 \leq b* \leq 35$, preferably in the range of $-30 \leq a* \leq 30$ or $-30 \leq b* \leq 30$, coloring can be brought close to a color friendly to human eyes without too strong tinting strength. Thereby, the present invention is especially suitable for use as a composition for coating or a transparent material for coloring ultraviolet protection.

(Metal element doping and factors of molar absorption coefficient increase)

Factors of increase of a molar absorption coefficient of the M2 doped oxide particles of the present invention by doping M2 into the oxide particles (M1Ox) are not certain. Primarily, absorption of a light by a substance is considered to be absorption of a light of a specific wavelength (light energy) based on an electronic transition according to the energy level specific to the substance. The present applicant believes that the factors of increase of light absorption efficiency against a same quantity of lights are as follows. By doping M2 to the oxide particle (M1Ox), strain of the crystal lattice may occurs, new bonds due to random combination of -M1-oxygen-M2- occur, or a defective site of oxygen or a defective site of M1 or M2 or another metal element or metalloid element, etc. occurs, which result in an increase in light absorption ability due to occurrence of an energy level not similar to the energy levels originally possessed by the oxide particles (an increase of the energy level number), and an increase in light absorption ability caused by enabling a light absorbed only in the vicinity of the surface layer of the particle to enter inside of the particle (an increase in light absorption efficiency of a material). These increases raise a molar absorption coefficient of the M2 doped oxide particles. These mechanisms are the factors of increase of light absorption efficiency against a same quantity of lights.

(Method of controlling color characteristic of M2 doped oxide particles: Modification treatment of a functional group or oxidation number)

In the present invention, it is also possible to control color characteristics in the visible region by modification treatment of a functional group contained in the M2 doped oxide particles having the above controlled molar ratio (M2/M1), or by modification treatment of oxidation number to a metal element or metalloid element (M1, M2, Mn). Regarding the modification of a functional group, a ratio of a functional group such as a hydroxyl group contained in the M2 doped oxide particles can be controlled by a method using a reaction such as a substitution reaction, addition reaction, elimination reaction, dehydration reaction, condensation reaction to a functional group contained in the M2 doped oxide particle. In controlling the ratio of a functional group such as a hydroxyl group, the ratio of the functional group may be increased or decreased, or a new functional group may be added. Examples of the modification treatment of a functional group include an oxidation or reduction reaction, an esterification reaction achieved by a dehydration and condensation reaction in which OH is removed from a carboxyl group (-COOH) and H is removed from a hydroxyl group (-OH), and another method in which hydrogen peroxide or ozone is allowed to act on the M2 doped oxide particles. Thereby, the ratio of a functional group such as a hydroxyl group contained in the M2 doped oxide particles can be controlled. Further, modification treatment of oxidation number can be also performed to a metal element or metalloid element (M1, M2, Mn) contained in M2 doped oxide particles. For example, in Co doped oxide particles (M2 = Co), modification treatment of oxidation number from Co (+II) to Co (+III) or vice versa can be performed. Further, when the M2 doped oxide particles are precipitated in a liquid, it is also possible to control a ratio of a functional group such as a hydroxyl group contained in the M2 doped oxide particles and to control the oxidation number to a metal element or metalloid element (M1, M2, Mn), by a method of precipitating the M2 doped oxide particles, or a method of controlling the pH, or the like. Further, as an example of a dehydration reaction in modification treatment of a functional group and modification treatment of oxidation number, it is possible by a heat treatment method of the M2 doped oxide particle to control a ratio of a functional group such as a hydroxyl group contained in the M2 doped oxide particle and to control an oxidation number of M1, M2, Mn.

(Preferable embodiment-1 of M2 doped oxide particles)

In the present invention, a primary particle diameter of the M2 doped oxide particles is preferably 1 nm or more and 100 nm or less, more preferably 1 nm or more and 50 nm or less. As described above, it is assumed that by constituting a composite oxide composed of M1 and M2 contained in the M2 doped oxide particles, a molar absorption coefficient and color characteristics of the M2 doped oxide particles can be controlled, and the surface of the particles can significantly affect these properties, and the like. Thereby, it is understood that the M2 doped oxide particles having a primary particle diameter of 100 nm or less have surface areas greater than the M2 doped oxide particles having a primary particle diameter of more than 100 nm, and that control of a molar ratio of M2 to M1 (M2/M1) in the M2 doped oxide particles greatly affects an average molar absorption coefficient and color characteristics of the M2 doped oxide particles. Therefore, in the M2 doped oxide particles having a primary particle diameter of 100 nm or less, it is an advantage that predetermined characteristics (particularly suitable characteristics for a composition for coating or a transparent material for coloring ultraviolet protection) can be exerted by controlling a molar ratio (M2/M1) of the M2 doped oxide particles.

(Preferable embodiment-2 of M2 doped oxide particles)

In the present invention, at least a part of the surface of the M2 doped oxide particles may be coated with a various compound. Examples of the compound include an aluminum compound such as aluminum oxide, a phosphorus or calcium compound such as calcium phosphate and apatite, a titanium compound such as titanium oxide, and a silicon compound such as silicon oxide. By these coatings, in addition to control of an average molar absorptivity coefficient of the present invention, control of color characteristics such as reflection characteristics, transmission characteristics and hue is possible. Control of an average molar absorption coefficient to the extent that is not possible by only the control method of the present invention, is also possible. Further, by optionally coating the surface of the particles, photocatalytic ability increased by increase of a molar absorption coefficient in the wavelength range of 200 nm to 380 nm, can be suppressed, and degradation and the like of a resin contained in a coated body or a skin of a human body due to photocatalytic ability can be suppressed. Further, it is an advantage that chemical stability such as water resistance, acid resistance and alkali resistance can be imparted to the M2 doped oxide particles by coating the surface of the M2 doped oxide particles with a compound. Regarding coated M2 doped oxide particles, a primary particle diameter of the M2 doped oxide particles themselves is also preferably 100 nm or less, more preferably 50 nm or less. In case that the M2 doped oxide particles at least a part of the surface of which is coated, constitute an aggregate of a plurality of the M2 doped oxide particles, a size of the aggregate is preferably 100 nm or less, more preferably 50 nm or less. In the present invention, in the M2 doped oxide particles at least a part of the surface of which is coated by a compound, it is preferable that the average primary particle diameter of the M2 doped oxide particles after coating is 100.5% or more and 190% or less relative to the average primary particle diameter of the M2 doped oxide particles before coating. When the compound coating on the M2 doped oxide particles is too thin, the effects regarding color characteristics of the compound coated M2 oxide particles and the like may not exhibit. Thus, it is preferable that the average primary particle diameter of the M2 doped oxide particles after coating by the compound is not less than 100.5% relative to the average primary particle diameter of the M2 doped oxide particles before coating. When the coating is too thick, or when a coarse aggregate is coated, control of color characteristics is difficult. Thus, it is preferable that the average primary particle diameter of the M2 doped oxide particles after coating by the compound is not more than 190% relative to the average primary particle diameter of the M2 doped oxide particles before coating. The M2 doped oxide particles coated with a compound of the present invention may be core/shell type M2 doped oxide particles in which the entire surface of the core M2 doped oxide particle is uniformly coated with the compound. The M2 doped oxide particles are preferably compound coated M2 doped oxide particles in which a plurality of the M2 doped oxide particles are not aggregated and at least a part of the surface of a single M2 doped oxide particle is coated with a compound. But, the M2 doped oxide particles may be M2 doped oxide particles in which at least a part of the surface of an aggregate in which a plurality of M2 doped oxide particles are aggregated, is coated with a compound. In the present invention, M2 doped oxide particles in which at least a part of the surface of the M2 doped oxide particles is coated with a silicon compound, have an advantage that an average molar absorption coefficient increase rate which is an increase rate of an average molar absorption coefficient, can be 120% or more.

A compound coating at least a part of the surface of the M2 doped oxide particles of the present invention is a compound containing an amorphous silicon oxide. By containing the amorphous silicon oxide as the silicon compound, a molar absorption coefficient of the M2 doped oxide particles can be further increased, and the color characteristics such as reflectivity, transmittance, hue, chroma and the like can be controlled more strictly.

(Method of producing M2 doped oxide particles: Preferable method-1)

As an example of a method of producing M2 doped oxide particles of the present invention, it is preferable to use a method of producing M2 doped oxide particles by providing an oxide raw material liquid containing at least a raw material of M2 doped oxide particles and an oxide precipitation solvent containing at least an oxide precipitating substance for precipitating M2 doped oxide particles, and precipitating M2 doped oxide particles by a method such as reaction, crystallization, precipitation and coprecipitation, in a mixed fluid in which the oxide raw material liquid and the oxide precipitation solvent are mixed. Since at least a part of the surface of the M2 doped oxide particles is coated with a compound containing an amorphous silicon oxide, it is preferable to use a method of producing M2 doped oxide particles by coating at least a part of the surface of the M2 doped oxide particles with a silicon compound as an example of a coating compound by mixing the mixed fluid containing the precipitated M2 doped oxide particles with a silicon compound raw material liquid containing at least a raw material of the silicon compound. M1 and M2 contained in the M2 doped oxide particles may be contained together in the oxide raw material liquid, or may be contained in the oxide raw material liquid and the oxide precipitation solvent respectively, or may be contained in both the oxide raw material liquid and the oxide precipitation solvent, or may be contained in both of the oxide raw material liquid and the oxide precipitation solvent or the silicon compound raw material liquid.

A raw material of M2 doped oxide particles of the present invention is not particularly limited. Any substances can be used as long as the substances become M2 doped oxide particles in a manner such as a reaction, crystallization, precipitation, coprecipitation or the like. In the present invention, hereinafter, a compound of M1 or M2 is referred to as a compound. The compound is not particularly limited, but includes, for example, a salt, an oxide, a hydroxide, a hydroxide oxide, a nitride, a carbide, a complex, an organic salt, an organic complex, an organic compound of the metal or metalloid including M1 or M2, or a hydrate thereof, an organic solvate thereof, and the like. It is also possible to use a simple substance of M1 or M2. A metal salt or metalloid salt is not particularly limited, but includes a nitrate, a nitrite, a sulfate, a sulfite, a formate, an acetate, a phosphate, a phosphite, a hypophosphite, a chloride, an oxy salt, an acetylacetonate of the metal or metalloid, or a hydrate thereof, an organic solvate thereof and the like. An organic compound includes a metal alkoxide, a metalloid alkoxide, and the like. These metal compound or metalloid compound may be used alone, or a mixture of a plurality of these compounds may be used as a raw material of oxide particles. In the present invention, a molar ratio of M2 to M1 (M2/M1) constituting the M2 doped oxide particles is 0.01 or more and 1.00 or less.

In case that the M2 doped oxide particles of the present invention are coated with a silicon compound, a raw material of the silicon compound includes a silicon oxide, a silicon hydroxide, other compounds such as a silicon salt and a silicon alkoxide, and a hydrate thereof. Not particularly limited, it includes silicates such as sodium silicate, phenyltrimethoxysilane, methyltrimethoxysilane, methyltriethoxysilane, 3-glycidoxypropyltrimethoxysilane, 3-trifluoropropyl-trimethoxysilane, methacryloxypropyltriethoxysilane, tetramethoxysilane (TMOS), tetraethoxysilane (TEOS), and an oligomeric condensate of TEOS, for example, ethyl silicate 40, tetraisopropylsilane, tetrapropoxysilane, tetraisobutoxysilane, tetrabutoxysilane, and a similar material thereof. Further as a raw material of silicon oxide, another siloxane compound, bis(triethoxysilyl)methane, 1,9-bis(triethoxysilyl)nonane, diethoxydichlorosilane, triethoxychlorosilane and the like may be used. These are not used only for coating surfaces of the particles, but also can be used as compounds containing the M1 or M2.

Further, when a raw material of M2 doped oxide particles or a raw material of a compound for coating is a solid, it is preferable to use each raw material in a molten state, or in a state of being mixed or dissolved in a solvent described below, including a dispersion state. Even when each raw material is a liquid or gas, it is preferable to use it in a state of being mixed or dissolved in a solvent described below, including a dispersion state.

An oxide precipitation substance is not particularly limited as long as the substance can make a raw material of M2 doped oxide particles contained in an oxide raw material liquid be precipitated as M2 doped oxide particles. For example, an acidic substance or a basic substance may be used. It is preferable to use an oxide precipitation substance at least in a state that the substance is mixed, dissolved or molecularly dispersed in a solvent described below.

A basic substance includes a metal hydroxide such as sodium hydroxide and potassium hydroxide, a metal alkoxide such as sodium methoxide and sodium isopropoxide, an amine compound such as triethylamine, diethylaminoethanol and diethylamine, ammonia and the like.

An acidic substance includes an inorganic acid such as aqua regia, hydrochloric acid, nitric acid, fuming nitric acid, sulfuric acid, fuming sulfuric acid, and an organic acid such as formic acid, acetic acid, chloroacetic acid, dichloroacetic acid, oxalic acid, trifluoroacetic acid, trichloroacetic acid, citric acid and the like. The basic substance and the acidic substance can also be used for precipitating M2 doped oxide particles or a compound for coating.

(Solvent)

A solvent used in preparation of an oxide raw material liquid and an oxide precipitation solvent, includes, for example, water, an organic solvent, or a mixed solvent of a plurality of these solvents. The water includes tap water, ion exchange water, pure water, ultrapure water, RO water (reverse osmosis water) and the like. The organic solvent includes, an alcohol solvent, an amide solvent, a ketone solvent, an ether solvent, an aromatic compound solvent, carbon disulfide, an aliphatic compound solvent, a nitrile solvent, a sulfoxide solvent, a halogen compound solvent, an ester solvent, an ionic liquid, a carboxylic acid compound, a sulfonic acid compound and the like. Each of the solvents may be used alone, or a plurality of them may be mixed and used. An alcohol solvent includes a monohydric alcohol such as methanol and ethanol, a polyol such as ethylene glycol and propylene glycol, and the like.

(Dispersing agent and the like)

In the present invention, various dispersing agents or surfactants may be used depending on a purpose or necessity, as long as they do not adversely affect production of M2 doped oxide particles. Not particularly limited, as a dispersing agent or a surfactant, various generally used commercial products or products, and newly synthesized products and the like may be used. As an example, a dispersing agent such as an anionic surfactant, a cationic surfactant, a nonionic surfactant, and various polymers and the like may be used. These may be used alone or two or more thereof may be used in combination. The surfactant and dispersing agent may be contained in at least one fluid of the oxide raw material liquid and oxide precipitation solvent. In addition, the surfactant and dispersing agent may be contained in another fluid different from the oxide raw material liquid and oxide precipitation solvent.

(Method of producing M2 doped oxide particles: Method outline-1)

In the present invention, as described above, firstly, at least M1 and M2 contained in the M2 doped oxide particles are preferably present together at least inside the particles. In producing M2 doped oxide particles by precipitation or the like, it is preferable to produce M2 doped oxide particles by precipitating oxides composed of a plurality of different elements (M1 and M2) at substantially the same time. For example, in the case that M2 doped oxide particles are precipitated by mixing an oxide raw material obtained by dissolving a zinc compound such as zinc nitrate hexahydrate as a raw material of zinc oxide (M1 = Zn) and a compound containing a metallic element or metalloid element for doping (M2) in an acidic aqueous solution, with an oxide precipitation solvent which is an aqueous solution of an alkali metal hydroxide such as sodium hydroxide (oxide precipitated substance), it is necessary to precipitate M2 doped oxide particles by mixing the oxide precipitation solvent having pH of 14 or more, to the oxide raw material liquid pH of about 1 to 2, preferably less than 1. Oxides composed of M1 or M2 contained in the oxide raw material liquid have respective different pHs, temperatures and the like suitable for their precipitation. For example, in the case that a basic oxide precipitation solvent is gradually added to an acidic oxide raw material liquid, pH of the mixed liquid of the oxide raw material liquid and the oxide precipitation solvent gradually changes from acidic to basic, and at first when the pH becomes close to the pH at which either one of M1 and M2 tends to precipitate, an oxide of the one of M1 or M2 precipitates or begins to precipitate, and later when the pH of the mixed liquid changes to the basic side by further adding the oxide precipitation solvent, the other oxide different from the earlier precipitated oxide precipitates. It may be understood that oxide particles composed of M1 and oxide particles composed of M2 precipitate step by step as explained above. In that case, it is difficult to prepare M2 doped oxide particles containing both M1 and M2 inside the particles. By instantaneously adjusting the pH of the mixed liquid to a pH at which both the oxide of M1 and the oxide of M2 precipitate, the apparent precipitation can be made simultaneously, so that at least premise conditions for producing M2 doped oxide particles containing both M1 and M2 inside the particles can be arranged.

(Method of producing M2 doped oxide particles: Method outline-2)

Further, when at least a part of the surface of the M2 doped oxide particles is coated with a silicon compound, it is preferable to coat before the M2 doped oxide particles aggregate. When as an example of a coating compound, a silicon compound raw material liquid is mixed with a fluid containing the M2 doped oxide particles, it is important after precipitating the M2 doped oxide particles, to precipitate the silicon compound on the surface of the M2 doped oxide particles by adding the silicon compound raw material liquid at a rate faster than aggregation occurs. Furthermore, by introducing the silicon compound raw material liquid into the fluid containing the M2 doped oxide particles, pH of the fluid containing the M2 doped oxide particles and a concentration of the silicon compound raw material gradually change, If the silicon compound for coating the surface of the particles precipitates after an easily dispersible state is changed to an easily aggregating state, there is a possibility that it becomes difficult to coat before aggregation and the characteristics of the present invention cannot be exhibited. It is preferable to actuate the silicon compound raw material contained in the silicon compound raw material liquid immediately after the M2 doped oxide particles precipitate.

(Method of producing M2 doped oxide particles: Apparatus)

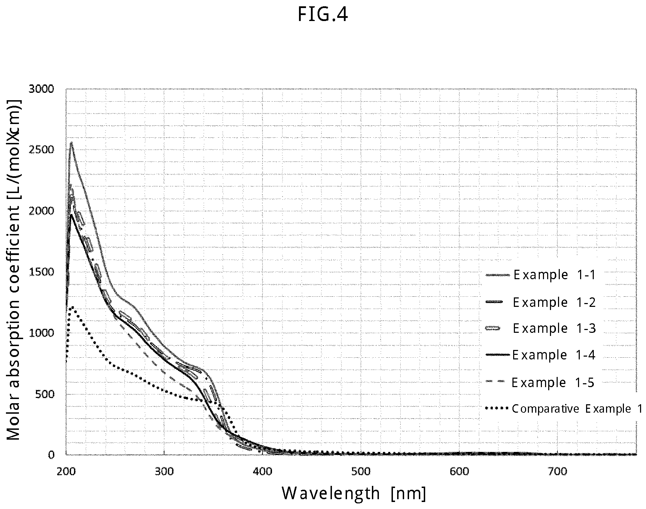

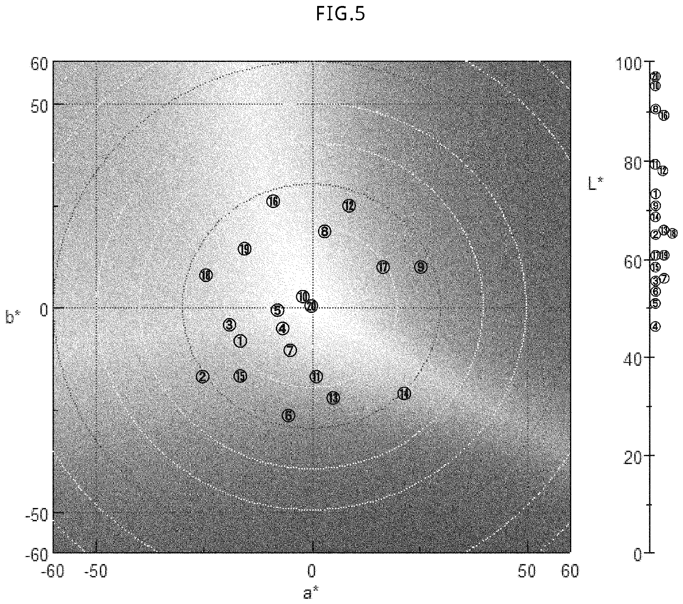

A method of producing M2 doped oxide particles of the present invention includes, for example, a method of producing M2 doped oxide particles by using a microreactor, or by a reaction in a dilute system in a batch vessel or the like, and the like. The apparatus and method as proposed by the present applicant and described in JP 2009-112892 may be also used for producing M2 doped oxide particles. The apparatus described in JP 2009-112892 comprises a stirring tank having an inner peripheral surface which cross-section is circular, and a mixing tool attached to the stirring tank with a slight gap to the inner peripheral surface of the stirring tank, wherein the stirring tank comprises at least two fluid inlets and at least one fluid outlet; from one of the fluid inlets, the first fluid to be processed containing one of the reactants among the fluids to be processed is introduced into the stirring tank; from one fluid inlet other than the above inlet, the second fluid to be processed containing one of reactants different from the above reactant is introduced into the stirring tank through a different flow path; at least one of the stirring tank and the mixing tool rotates at a high speed relative to the other to let the above fluids be in a state of a thin film; and in the above thin film, the reactants contained in the first and second fluids to be processed are reacted. JP 2009-112892 further describes that three or more inlet tubes may be provided as shown in FIG 4 and 5 to introduce three or more fluids to be processed into the stirring tank. As an example of the microreactor, an apparatus using the same principle as the fluid processing apparatus described in Patent Literatures 6 and 7 can be used. Alternatively, M2 doped oxide particles may be prepared by using a pulverization method such as a beads mill and the like, and then a process of coating M2 doped oxide particles may be performed in a reaction vessel or the microreactor described above or the like.

(Composition for coating or composition for a transparent material-1)

The coloring ultraviolet protection agent of the present invention is intended for protection of ultraviolet rays and coloring, and as one example, it is used for a composition for coloring or a composition for a transparent material. The composition for coating is not particularly limited, and examples thereof include a composition for coating for use in various paints such as a solvent based paint and water based paint, and a composition for coating intended for a cosmetic such as a lipstick and foundation, sunscreen and the like, or for application to a skin. The composition for a transparent material includes a composition for use in a coated body required to have transparency, a glass for use in a building or vehicle or a glass for eyeglasses, a transparent resin or a film like composition, and a composition contained in a glass, transparent resin or clear coating film, a composition contained in an intermediate film of a combined glass, a film like composition used for a film combined with a glass, such as one attaching to a glass or transparent resin, a paint for coating on a glass, and the like. The above transparent resin includes PMMA (polymethyl methacrylate), PC (polycarbonate), PET (polyethylene terephthalate), and the like.

(Composition for coating or composition for a transparent material-2)

When used as a paint, coating film, a cosmetic or the like, or a material of a glass or transparent resin of a composition for coating or a composition for a transparent material, by using a method of mixing the M2 doped oxide particles of the coloring ultraviolet protection agent of the present invention to a composition such as a coating film forming a paint or coated body or a cosmetic or the like, or a method of directly kneading them to a glass or pre-hardened glass or clear resin, or a method of mixing them in a film for various glasses or a composition for forming a clear coating film, a composition for coating for coloring ultraviolet protection or a composition for a transparent material for coloring ultraviolet protection suitable for effectively shielding ultraviolet rays and coloring according to the purpose can be obtained. The above composition for coating for coloring ultraviolet protection or the composition for a transparent material for coloring ultraviolet protection, if necessary, may further comprise an additive such as a pigment, dye, wetting agent, dispersing agent, color separation inhibitor, leveling agent, viscosity modifier, anti-skinning agent, anti-gelling agent, antifoaming agent, thickener, anti-sagging agent, antifungal agent, ultraviolet absorber, film-forming assistant agent, surfactant, resin component, if necessary, depending on its purpose. A resin component for painting purpose or for the purpose of forming an intermediate film for adhesion between glasses in a film like form, includes polyester resins, melamine resins, phenol resins, epoxy resins, vinyl chloride resins, acrylic resins, urethane resins, silicone resins, fluorine resins and the like. A coating material which a paint containing the coloring ultraviolet protection agent of the present invention is applied to, may be a single coating material composed of a single layer composition for coating, or a multilayer coating material composed of a plurality of a composition for coating such as laminated coating film as described in JP 2014-042891 or JP 2014-042892. The coating material may be performed by adding it to a paint containing a pigment, or to a paint such as a clear paint. In the case where the above film like composition is aimed, if necessary, a binder resin, curing agent, curing catalyst, leveling agent, surfactant, silane coupling agent, defoaming agent, coloring agent such as a pigment or dye, antioxidant and the like may be contained.

(Composition for coating or composition for a transparent material-3)

The composition for coating for coloring ultraviolet protection or the composition for a transparent material for coloring ultraviolet protection comprises powers of M2 doped oxide particles; a dispersion wherein M2 doped oxide particles are dispersed in a liquid dispersion medium; and a dispersion wherein M2 doped oxide particles are dispersed in a solid such as glass and resin, and the like. M2 doped oxide particles contained in the above composition for a transparent material may be composed of one M2 doped oxide particle, or may be composed of an aggregate of a plurality of M2 doped oxide particles, or may be composed of both of those. When M2 doped oxide particles are composed of an aggregate of a plurality of M2 doped oxide particles, a size of the aggregate is preferably 100 nm or less. Further, the composition for coating for coloring ultraviolet protection or the composition for a transparent material for coloring ultraviolet protection may be used together with various coloring materials, or may be a composition for overcoating on a glass as a coating film. Further, in the case where a composition for coating for coloring ultraviolet protection or a composition for a transparent material for coloring ultraviolet protection is a dispersion, a dispersion medium includes water such as tap water, distilled water, RO water (reverse osmosis water), pure water and ultrapure water; an alcohol solvent such as methanol, ethanol and isopropyl alcohol; a polyhydric alcohol solvent such as propylene glycol, ethylene glycol, diethylene glycol and glycerine; an ester solvent such as ethyl acetate and butyl acetate; an aromatic solvent such as benzene, toluene and xylene; a ketone solvent such as acetone and methyl ethyl ketone; a nitrile solvent such as acetonitrile; silicone oil, a vegetable oil, a wax and the like. These may be used alone or two or more thereof may be used in combination.

(Color of composition for coating or composition for a transparent material)

A color of a coating material used for the composition for coating for coloring ultraviolet protection or the composition for a transparent material for coloring ultraviolet protection of the present invention, or a transparent material such as a film, glass and the like used for the composition for a transparent material for coloring ultraviolet protection, is not particularly limited, and the composition for coating for coloring ultraviolet protection or a composition for a transparent material for coloring ultraviolet protection of the present invention can be used for a desired hue. Further, since color characteristics can be strictly and accurately controlled by controlling the molar ratio (M2/M1) of the M2 doped oxide particles of the present invention, it is also preferable to use it even as a composition for coloring for coloring ultraviolet protection. The composition for coating, for a transparent material or for coloring of the present invention contains the M2 doped oxide particles, so that ultraviolet protection ability can be increased when the composition is used as a paint or coated material such as a coated body used for a building, vehicle, etc., or when the composition is used for as a transparent material of a film like composition such as a clear coating film or a glass or a display or a contact lens; decomposition of an organic substance contained in a coated body such as a building and vehicle, or damages of a skin in a human body and the like can be suppressed; damages of an organic compound and equipment in a room by ultraviolet rays transmitted through a glass used for a building or vehicle and the like, can be suppressed; in addition, improvement of transparency of a glass, clear coating film and the like can be contributed due to reduction of an amount of usage and thereby high transmission characteristics; and, the aesthetic appearance, texture or designability can be enhanced due to strict control of color characteristics such as a hue.

(Color of composition for coating, composition for a transparent material or composition for coloring)

Regarding a color of the coating material or transparent material, M2 doped oxide particles may be preferably blended to a composition for coating used for a coated body having a white color, gray color or black color such as color of white color of a lightness of 10 to black color of a lightness 0 in the Munsell color system, a red color such as color having a hue from RP to YR in the Munsell hue circle; a yellow to green color such as a color having a hue from Y to BG in the Munsell hue circle; a blue to purple color such as a color having a hue from B to P in the Munsell hue circle (each of these colors includes a metallic color) may be blended to a composition for coating used for a coating material. However, the present invention is not limited to these colors, and may be a color of any other hues. In addition, by using a composition for coating or a composition for a transparent material containing the M2 doped oxide particles of the present invention to a top coat of a coating film or coated body exhibiting these colors, impairment of coloring of each color can be remarkably reduced, and effective coloring is also possible, so that it is also suitable as a composition for coloring for enhancing designability of a coated body. As a pigment or dye optionally included in a composition for coating, a transparent material or coloring, various pigments and dyes may be used, and for example, all pigments and dyes registered in the color index may be used. Among these colors, a pigment or dye constituting a pigment constituting a green color includes, for example, a pigment or dye classified into C. I. Pigment Green; a pigment constituting a blue color includes, for example, a pigment or dye classified into C. I. Pigment Blue; a pigment constituting a white color includes, for example, a pigment or dye classified into C. I. Pigment White; a pigment constituting a yellow color includes, for example, a pigment or dye classified into C. I. Pigment Yellow; a red color includes, for example, a pigment or dye classified into C. I. Pigment Red in the Color Index, a pigment or dye classified into C. I. Pigment Violet or C. I. Pigment Orange in the Color Index, and the like. More specific examples include a quinacridone pigment such as C. I. Pigment Red 122 and C. I. Violet 19; a diketopyrrolopyrrole pigment such as C. I. Pigment Red 254 and C. I. Pigment Orange73; a naphthol pigment such as C. I. Pigment Red 150 and C. I. Pigment Red 170; a perylene pigment such as C. I. Pigment Red 123 and C. I. Pigment Red 179; and an azo pigment such as C. I. Pigment Red 144, and the like. These pigments and dyes may be used alone, or a plurality of these may be mixed and used. The composition comprising M2 doped oxide particles of the present invention may be also mixed in a composition for coating, a transparent material or coloring alone without mixing with the above pigments and dyes and the like.

EXAMPLE

Hereinafter, the present invention is explained in more detail with reference to examples, but the present invention is not limited only to these examples. Pure water having conductivity of 0.80 μS/cm (measurement temperature: 20 °C) was used for pure water in the following examples, unless otherwise noted.

(Preparation of TEM observation sample and preparation of STEM observation sample)

A part of the wet cake samples of the M2 doped oxide particles obtained in Examples was dispersed in propylene glycol, and further was diluted to 100 fold by isopropyl alcohol (IPA). The resulting diluted liquid was dropped to a collodion membrane or a micro grid, and dried to prepare a TEM observation sample or an STEM observation sample.

(Transmission electron microscopy and energy dispersive X-ray analyzer: TEM-EDS analysis)

For observation and quantitative analysis of the M2 doped oxide particles by TEM-EDS analysis, the transmission electron microscopy JEM-2100 (JEOL Ltd.) equipped with the energy dispersive X-ray analyzer JED-2300 (JEOL Ltd.) was used. The observation condition was the acceleration voltage of 80 kV, and the observation magnification of 25,000 times or more. The particle diameters were calculated from the maximum distance between two points on the outer periphery of the M2 doped oxide particles observed by TEM, and the average value of the measured particle diameters of 100 particles (average primary particle diameter) was calculated. A molar ratio of the elemental components contained in the silicon compound coated metal element doped metal oxide was calculated by TEM-EDS, and the average value of the results of calculated molar ratio for 10 or more particles was calculated.

(Scanning transmission electron microscopy and energy dispersive X-ray analyzer: STEM-EDS analysis)

For the mapping and quantification of elements contained in the M2 doped oxide particles by STEM-EDS analysis, the atomic resolution analytical electron microscopy JEM-ARM200F (JEOL Ltd.) equipped with the energy dispersive X-ray analyzer Centurio (JEOL Ltd.) was used. The observation condition was the acceleration voltage of 80 kV and the observation magnification of 50,000 times or more, and a beam diameter of 0.2 nm was used for analysis.

(X-ray diffraction measurement)

For the X-ray diffraction (XRD) measurement, the powder X-ray diffractometer Empyrean (Spectris Co., Ltd., PANalytical Division) was used. The measurement condition was measurement range of 10 to 100 [°2Theta], Cu anticathode, tube voltage of 45 kV, tube current of 40 mA, and scanning speed of 0.3 °/min. The XRD was measured using the dry powder of the M2 doped oxide particles obtained in each Example.

(Hue and chroma using absorption spectrum and reflection spectrum measurement)

Absorption spectrum, L* value, a* value, b* value, hue, and chroma were measured by ultraviolet visible near infrared spectroscopy (product name: V-770, JASCO Corporation). Measurement range of absorption spectrum was 200 nm to 800 nm, the sampling rate was 0.2 nm, and the measurement speed was low speed. After measuring absorption spectrum, a molar absorption coefficient at each measurement wavelength was calculated from the absorbance obtained from the measurement result and the concentration of M2 doped oxide particles in the dispersion, and the graph was prepared showing the measurement wavelength on the horizontal axis and the molar absorption coefficient on vertical axis. A liquid cell of thickness of 1 cm was used for measurements. Also, the molar absorption coefficients measured at a plurality of wavelengths from 200 nm to 380 nm were simply averaged so that the average molar absorption coefficient was calculated.
Measurement range of reflection spectrum was 200 to 2,500 nm, and the sampling rate was 2.0 nm, and the measurement speed was medium speed, and measurement method was a double beam photometry. Total reflection measurement for measuring specular reflection and diffuse reflection was performed. For a background measurement (baseline) in measuring powders, the standard white plate (product name: Spectralon™, Labsphere Inc.) was used. Reflection spectrum was measured using dry powders of the M2 doped oxide particles in each Example. Hue and chroma were measured from the reflection spectrum measurement result in the L*a*b* color system, in which the field of view was 2 (deg), the light source was D65-2, the color matching function was JIS Z 8701: 1999, and the data interval as 5 nm. Hue and chroma were calculated by the following equations from the obtained values in the L*a*b* color system: hue H = b*/a*, and chroma C = ((a*)$^2$ + (b*)$^2$)$^{1/2}$.

(Reference Example 1)

As M2 doped oxide particles of Example 1, described are M2 doped zinc oxide particles wherein M1 is zinc, zinc oxide is doped with cobalt, manganese, iron, magnesium or cobalt and aluminum as M2 (Co-ZnO, Mn-ZnO, Fe-ZnO, Mg-ZnO, (Co+Al)-ZnO). An oxide raw material liquid (liquid A), an oxide precipitation solvent (liquid B), and a silicon compound raw material liquid (liquid C) in case of coating at least a part of the particle surface with a silicon compound, were prepared using the high-speed rotary dispersion emulsification apparatus CLEAMIX (product name: CLM-2.2 S, M. Technique Co., Ltd.). Specifically, based on the formulation of the oxide raw material liquid shown in Example 1 of Table 1, the components of the oxide raw material liquid were mixed homogeneously by stirring using CLEARMIX at preparation temperature of 40 °C and at the rotor rotational speed of 20,000 rpm for 30 min to prepare the oxide raw material liquid. Based on the formulation of the oxide precipitation solvent shown in Example 1 of Table 1, the components of the oxide precipitation solvent were mixed homogeneously by stirring using CLEARMIX at preparation temperature of 45 °C and at the rotor rotational speed of 15,000 rpm for 30 min to prepare the oxide precipitation solvent. Based on the formulation of the silicon compound raw material liquid shown in Example 1 of Table 1, the components of the silicon compound raw material liquid were mixed homogeneously by stirring using CLEARMIX at preparation temperature of 20 °C and at the rotor rotational speed of 6,000 rpm for 10 min to prepare the silicon compound raw material liquid. Regarding the substances represented by the chemical formula and abbreviations set forth in Table 1, $Zn(NO_3)_2 \cdot 6H_2O$ is zinc nitrate hexahydrate (Kanto Kagaku Co., Ltd.), $Co(NO_3)_2 \cdot 6H_2O$ is cobalt nitrate hexahydrate (Kanto Kagaku Co., Ltd.), $Mn(NO_3)_2 \cdot 6H_2O$ is manganese nitrate hexahydrate (Kanto Kagaku Co., Ltd.), $Al(NO_3)_3 \cdot 9H_2O$ is aluminum nitrate nonahydrate (Kanto Kagaku Co., Ltd.), $Fe(NO_3)_3 \cdot 9H_2O$ is iron nitrate nonahydrate (Kanto Kagaku Co., Ltd.), $Mg(NO_3)_2 \cdot 6H_2O$ is magnesium nitrate hexahydrate (Kanto Kagaku Co., Ltd.), TEOS is tetraethyl orthosilicate (Wako Pure Chemical Industry Ltd.), EG is ethylene glycol (Kishida Chemical Co., Ltd.), MeOH is methanol (Godo Co., Ltd.), and NaOH is sodium hydroxide (Kanto Chemical Co., Inc.).

Table 2 shows the operating conditions of the fluid treatment apparatus, and the average primary particle diameters calculated from the TEM observation result of the M2 doped zinc oxide particles, and the molar ratios (M2/M1) calculated from TEM-EDS analysis, and the calculated values calculated from the formulations and introduction flow rates of liquid A, liquid B and liquid C. Here, [calculated value] of the M2 doped oxide particles in the molar ratio (M2/M1) shown in Table 2, is the result calculated from the molar concentrations of M1 and M2 contained in liquid A. [EDS] is the average value of the result of the molar ratio (M2/M1) of the elemental components constituting the particles calculated by the above TEM-EDS. The introduction temperatures (liquid sending temperatures) and the introduction pressures (liquid sending pressures) of liquid A, liquid B and liquid C shown in Table 2 were measured using a thermometer and a pressure gauge provided in a sealed inlet path leading to the space between the processing surfaces 1 and 2 (the first introduction part d1, the second introduction part d2 and the third introduction part d3). The introduction temperature of liquid A shown in Table 2 is the actual temperature of liquid A under the introduction pressure in the first introduction part d1. Similarly, the introduction temperature of liquid B shown in Table 2 is the actual temperature of liquid B under the introduction pressure in the second introduction part d2. The introduction temperature of liquid C shown in Table 2 is the actual temperature of liquid C under the introduction pressure in the third introduction part d3.

For the pH measurement, the pH meter, model number D-51 manufactured by HORIBA Ltd. was used. The pH of liquid A and liquid B were measured at room temperature prior to introduction into the fluid processing apparatus. Further, it is difficult to measure the pH of the mixed fluid immediately after mixing the oxide raw material liquid and the oxide precipitation solvent, and the pH of the mixed fluid immediately after mixing the fluid prepared after mixing the oxide raw material liquid and the oxide precipitation solvent and the silicon compound raw material liquid. Therefore, the M2 doped oxide particle dispersion liquid was discharged from the apparatus and collected in a beaker b, and the pH of the liquid was measured at room temperature.

Dry powders and wet cake samples were produced from the M2 doped oxide particle dispersion liquid which was discharged from the fluid processing apparatus, and collected in the beaker. The manufacturing method was conducted according to a conventional method of this type of processing. The discharged M2 doped oxide particle dispersion liquid was collected, and the M2 doped oxide particles were settled, and the supernatant was removed. Thereafter, the M2 doped oxide particles were washed and settled three times repetitively with 100 parts by weight of pure water, and then, were washed and settled three times repetitively with pure water. A part of the finally obtained wet cake of the M2 doped oxide particles was dried at 25 °C at -0.10 MPaG for 20 hours to obtain the dry powders. The rest was the wet cake sample. The M2 doped zinc oxide particles obtained in Example 1-1, Example 1-9 and Example 1-10 were subjected with heat treatment using an electric furnace to change color characteristics. The powders of Example 1-1 were heated at 200 °C (Example 1-18), and 300 °C (Example 1-19). The powders of Example 1-9 were heated at 300 °C (Example 1-16). The powders of Examples 1-10 were heated at 300 °C (Example 1-17). The every heat treatment time was 30 minutes.

As shown in Tables 1 and 2, in Comparative Example 1, zinc oxide particles not doped with M2 were prepared.

[Table 1]

| | | Formulation of the 1st fluid (liquid A) Oxide raw material liquid | | | | | | Formulation of the 2nd fluid (liquid B) Oxide precipitation solvent | | | | Formulation of the 3rd fluid (liquid C) | | | | |
| | | Formulation | | | | pH | | Formulation | | pH | | Formulation | | | pH | |
| | | Raw material [wt%] | Raw material [wt%] | Raw material [wt%] | Raw material [wt%] | pH | [°C] | Raw material [wt%] | Raw material [wt%] | pH | [°C] | Raw material [wt%] | Raw material [wt%] | Raw material [wt%] | pH | [°C] |
|---|---|---|---|---|---|---|---|---|---|---|---|---|---|---|---|---|
| Example | 1-1 | Zn(NO$_3$)$_2$·6H$_2$O [3.000 wt%] | Co(NO$_3$)$_2$·6H$_2$O [0.045 wt%] | - [0.000 wt%] | EG [96.955 wt%] | 4.57 | 22.3 | NaOH [9.0 wt%] | Pure water [91.0 wt%] | >14 | - | - | - | - | - | - |
| | 1-2 | Zn(NO$_3$)$_2$·6H$_2$O [3.000 wt%] | Co(NO$_3$)$_2$·6H$_2$O [0.246 wt%] | - [0.000 wt%] | EG [96.754 wt%] | 4.10 | 22.0 | NaOH [9.0 wt%] | Pure water [91.0 wt%] | >14 | - | - | - | - | - | - |
| | 1-3 | Zn(NO$_3$)$_2$·6H$_2$O [3.000 wt%] | Co(NO$_3$)$_2$·6H$_2$O [0.325 wt%] | - [0.000 wt%] | EG [96.675 wt%] | 3.84 | 22.2 | NaOH [9.0 wt%] | Pure water [91.0 wt%] | >14 | - | - | - | - | - | - |
| | 1-4 | Zn(NO$_3$)$_2$·6H$_2$O [3.000 wt%] | Co(NO$_3$)$_2$·6H$_2$O [0.978 wt%] | - [0.000 wt%] | EG [96.022 wt%] | 3.45 | 27.0 | NaOH [9.0 wt%] | Pure water [91.0 wt%] | >14 | - | - | - | - | - | - |
| | 1-5 | Zn(NO$_3$)$_2$·6H$_2$O [2.000 wt%] | Co(NO$_3$)$_2$·6H$_2$O [1.957 wt%] | - [0.000 wt%] | EG [96.043 wt%] | 3.40 | 25.0 | NaOH [9.0 wt%] | Pure water [91.0 wt%] | >14 | - | - | - | - | - | - |
| | 1-6 | Zn(NO$_3$)$_2$·6H$_2$O [3.000 wt%] | Co(NO$_3$)$_2$·6H$_2$O [0.445 wt%] | TEOS [0.180 wt%] | EG [96.375 wt%] | 3.84 | 22.2 | NaOH [9.0 wt%] | Pure water [91.0 wt%] | >14 | - | - | - | - | - | - |
| | 1-7 | Zn(NO$_3$)$_2$·6H$_2$O [3.000 wt%] | Co(NO$_3$)$_2$·6H$_2$O [0.978 wt%] | TEOS [0.140 wt%] | EG [95.882 wt%] | 3.45 | 27.0 | NaOH [9.0 wt%] | Pure water [91.0 wt%] | >14 | - | - | - | - | - | - |
| | 1-8 | Zn(NO$_3$)$_2$·6H$_2$O [3.000 wt%] | Fe(NO$_3$)$_3$·9H$_2$O [0.143 wt%] | - [0.000 wt%] | EG [96.857 wt%] | <1 | - | NaOH [9.0 wt%] | Pure water [91.0 wt%] | >14 | - | - | - | - | - | - |
| | 1-9 | Zn(NO$_3$)$_2$·6H$_2$O [3.000 wt%] | Fe(NO$_3$)$_3$·9H$_2$O [0.143 wt%] | Mn(NO$_3$)$_2$·6H$_2$O [0.044 wt%] | EG [96.813 wt%] | 2.78 | 31.1 | NaOH [9.0 wt%] | Pure water [91.0 wt%] | >14 | - | - | - | - | - | - |
| | 1-10 | Zn(NO$_3$)$_2$·6H$_2$O [3.000 wt%] | Mg(NO$_3$)$_2$·6H$_2$O [0.042 wt%] | - [0.000 wt%] | EG [96.958 wt%] | 3.67 | 31.6 | NaOH [9.0 wt%] | Pure water [91.0 wt%] | >14 | - | - | - | - | - | - |
| | 1-11 | Zn(NO$_3$)$_2$·6H$_2$O [2.000 wt%] | Co(NO$_3$)$_2$·6H$_2$O [0.193 wt%] | Al(NO$_3$)$_3$·9H$_2$O [2.273 wt%] | EG [95.534 wt%] | 0.24 | 22.2 | NaOH [9.0 wt%] | Pure water [91.0 wt%] | >14 | - | - | - | - | - | - |
| | 1-12 | Zn(NO$_3$)$_2$·6H$_2$O [3.000 wt%] | Fe(NO$_3$)$_3$·9H$_2$O [0.172 wt%] | - [0.000 wt%] | EG [96.828 wt%] | <1 | - | NaOH [9.0 wt%] | Pure water [91.0 wt%] | >14 | - | - | - | - | - | - |
| | 1-13 | Zn(NO$_3$)$_2$·6H$_2$O [1.840 wt%] | Co(NO$_3$)$_2$·6H$_2$O [0.597 wt%] | Al(NO$_3$)$_3$·9H$_2$O [1.550 wt%] | EG [96.013 wt%] | 0.57 | 16.4 | NaOH [9.0 wt%] | Pure water [91.0 wt%] | >14 | - | - | - | - | - | - |
| | 1-14 | Zn(NO$_3$)$_2$·6H$_2$O [1.840 wt%] | Co(NO$_3$)$_2$·6H$_2$O [0.289 wt%] | Al(NO$_3$)$_3$·9H$_2$O [1.948 wt%] | EG [95.923 wt%] | 0.54 | 16.3 | NaOH [9.0 wt%] | Pure water [91.0 wt%] | >14 | - | - | - | - | - | - |
| | 1-15 | Zn(NO$_3$)$_2$·6H$_2$O [3.000 wt%] | Co(NO$_3$)$_2$·6H$_2$O [0.325 wt%] | - [0.000 wt%] | EG [96.675 wt%] | 3.84 | 22.2 | NaOH [9.0 wt%] | Pure water [91.0 wt%] | >14 | - | TEOS [0.57 wt%] | MeOH [2.43 wt%] | EG [97.00 wt%] | 6.13 | 16.1 |
| Comparative Example 1 | | Zn(NO$_3$)$_2$·6H$_2$O [3.000 wt%] | - [0.000 wt%] | - [0.000 wt%] | EG [97.000 wt%] | 2.41 | 32.1 | NaOH [9.0 wt%] | Pure water [91.0 wt%] | >14 | - | - | - | - | - | - |

[Table 2]

EP 3 467 071 B1

18

| | | Introduction flow rate (liquid sending flow rate) [ml/min] | | | Introduction temperature (liquid sending temperature) [°C] | | | Introduction pressure (liquid sending pressure) [MPaG] | | | Discharged liquid | | Molar ratio (M2/M1) | | | | Average primary particle diameter |
|---|---|---|---|---|---|---|---|---|---|---|---|---|---|---|---|---|---|
| | | | | | | | | | | | pH | Temperature [°C] | M2 | M1 | Metal oxide doped zinc oxide particles | | [nm] |
| | | Liquid A | Liquid B | Liquid C | Liquid A | Liquid B | Liquid C | Liquid A | Liquid B | Liquid C | | | | | [Calcurated value] | [EDS] | |
| Example | 1-1 | 400 | 45 | - | 160 | 87 | - | 0.103 | 0.10 | - | 11.87 | 28.1 | Co | Zn | 0.015 | 0.015 | 7.86 |
| | 1-2 | 400 | 45 | - | 161 | 87 | - | 0.101 | 0.10 | - | 11.75 | 32.5 | Co | Zn | 0.084 | 0.084 | 7.88 |
| | 1-3 | 400 | 45 | - | 160 | 87 | - | 0.093 | 0.10 | - | 11.86 | 26.5 | Co | Zn | 0.111 | 0.111 | 7.76 |
| | 1-4 | 400 | 50 | - | 160 | 86 | - | 0.087 | 0.10 | - | 11.62 | 19.7 | Co | Zn | 0.333 | 0.333 | 7.69 |
| | 1-5 | 400 | 50 | - | 161 | 86 | - | 0.087 | 0.10 | - | 11.78 | 19.9 | Co | Zn | 1.000 | 1.000 | 7.89 |
| | 1-6 | 400 | 45 | 100 | 160 | 87 | 88 | 0.088 | 0.10 | 0.10 | 11.12 | 19.7 | Co+Si | Zn | 0.237 | 0.237 | 7.69 |
| | 1-7 | 400 | 45 | 100 | 160 | 87 | 88 | 0.086 | 0.10 | 0.10 | 7.85 | 17.3 | Co+Si | Zn | 0.401 | 0.401 | 7.94 |
| | 1-8 | 400 | 40 | - | 160 | 89 | - | 0.092 | 0.10 | - | 11.84 | 21.9 | Fe | Zn | 0.035 | 0.035 | 7.89 |
| | 1-9 | 400 | 40 | - | 163 | 89 | - | 0.089 | 0.10 | - | 11.65 | 29.0 | Fe+Mn | Zn | 0.050 | 0.050 | 7.94 |
| | 1-10 | 400 | 40 | - | 161 | 89 | - | 0.088 | 0.10 | - | 11.80 | 23.6 | Mg | Zn | 0.015 | 0.015 | 7.88 |
| | 1-11 | 400 | 40 | - | 163 | 89 | - | 0.099 | 0.10 | - | 9.85 | 26.6 | Co+Al | Zn | 1.000 | 1.000 | 7.68 |
| | 1-12 | 400 | 40 | - | 163 | 89 | - | 0.092 | 0.10 | - | 11.48 | 28.6 | Fe | Zn | 0.042 | 0.042 | 7.61 |
| | 1-13 | 400 | 30 | - | 164 | 90 | - | 0.101 | 0.10 | - | 7.65 | 26.6 | Co+Al | Zn | 1.000 | 1.000 | 7.84 |
| | 1-14 | 400 | 40 | - | 163 | 89 | - | 0.099 | 0.10 | - | 7.16 | 26.9 | Co+Al | Zn | 1.000 | 1.000 | 7.91 |
| | 1-15 | 400 | 40 | 100 | 160 | 87 | 88 | 0.089 | 0.10 | 0.10 | 10.62 | 17.9 | Co | Zn | 0.111 | 0.111 | 7.77 |
| Comparative Example 1 | | 400 | 38 | 100 | 140 | 90 | 90 | 0.297 | 0.10 | 0.10 | 12.49 | 28.1 | - | - | - | - | 7.92 |

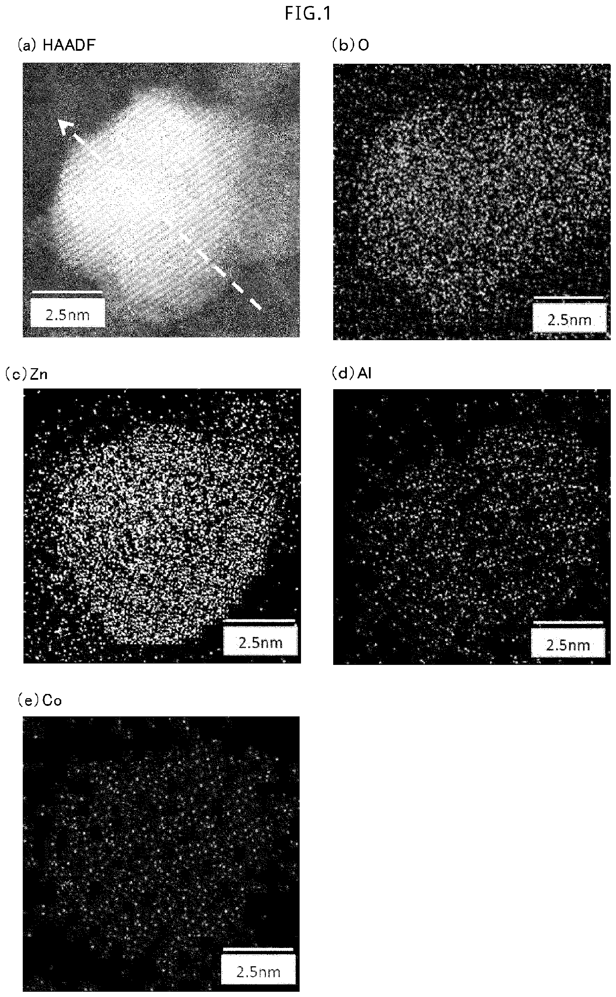

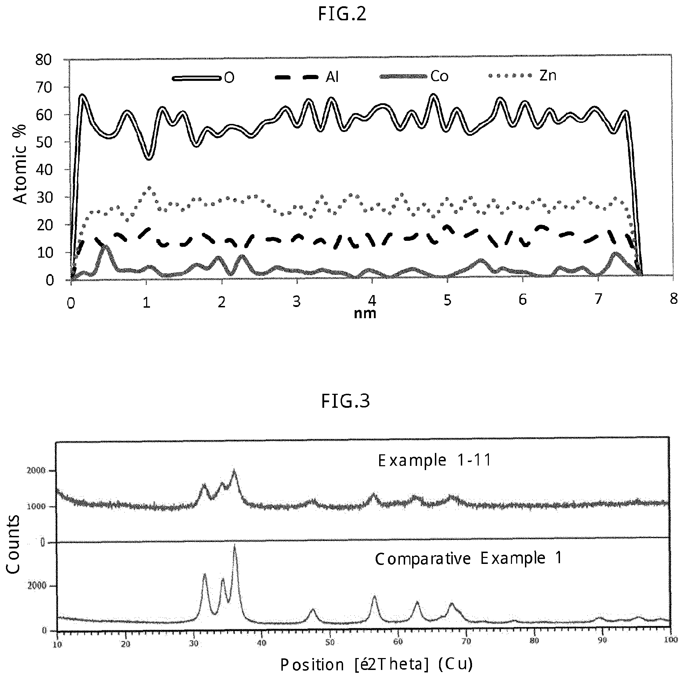

FIG 1 shows STEM mapping results of the cobalt aluminum doped zinc oxide particles obtained in Example 1-11. (a) shows a mapping result of a dark-field (HAADF), (b) shows a mapping result of oxygen (O), (c) shows a mapping result of zinc (Zn), (d) shows a mapping result of aluminum (Al), and (e) shows a mapping result of cobalt (Co). As seen from the HAADF image and the mapping results, it is understood that all elements are detected throughout the particles. Zinc, aluminum and cobalt are randomly detected in the range of substantially the same particle size, and it is considered that a solid solution oxide of zinc, aluminum and cobalt is formed. FIG 2 shows the result of a linear analysis at the position indicated by the broken line in the HAADF image of FIG 1, which shows the atomic% (mol%) of the elements detected in the line part from the edge to the other edge of the particle. As seen in FIG 2, all elements were detected from end to end in the analysis range in the linear analysis. Regarding cobalt, since the atomic% of Co contained in the whole particle was lower than those of the other elements, undetected parts (atomic% = 0) were also observed, but it is considered that Co should have been also detected throughout the particle. Further, in all of Examples 1-1 to 1-15, STEM mapping and linear analysis results similar to those of Example 1-11 were obtained. Furthermore, in Example 1-15, since the surface of the particle was coated with the silicon compound, silicon and oxygen were detected on the outer side of the solid solution oxide of zinc and cobalt. It was found that the particle was a particle in which the surface of the cobalt doped zinc oxide particle was coated with a silicon compound. Since the zinc oxide particles of Comparative Example 1 were not doped with M2, in the STEM mapping and linear analysis, a similar particle to those in Example 1-1 to Example 1-15 was observed except that M2 was not detected.

FIG 3 shows an XRD measurement result of the cobalt aluminum doped zinc oxide particles obtained in Example 1-11 and an XRD measurement result of the zinc oxide particles obtained in Comparative Example 1. As seen from FIG 3, in both the XRD measurement results of the cobalt aluminum doped zinc oxide particles obtained in Example 1-11 and the zinc oxide particles obtained in Comparative Example 1, a peak which can be identified as zinc oxide (ZnO) were detected, but the peak in Example 1-11 was detected as a broader peak as compared with one in Comparative Example 1. It is considered that cobalt and aluminum were incorporated into the particle, so that the strain in zinc oxide crystals may have occurred. Furthermore, for the particles obtained in Example 1-1 to Example 1-15, similar XRD measurement results to one in Example 1-11 were obtained.

FIG 4 shows a graph of molar absorption coefficients in the measurement wavelengths, which were calculated from the absorption spectrum of dispersions obtained by dispersing in propylene glycol the cobalt doped zinc oxide particles obtained in Examples 1-1 to 1-5 and the zinc oxide particles obtained in Comparative Example 1, and molar concentrations of the cobalt doped zinc oxide particles in the measured dispersions (converted as $ZnO + CoO$ (M2)) and the zinc oxide particles in the measured dispersions (converted as ZnO). As seen from FIG 4, it is found that the molar absorption coefficient of the cobalt doped zinc oxide particles in the wavelength range of 200 nm to 380 nm is improved compared with the same molar absorption coefficient of the zinc oxide particles of Comparative Example 1. Also, Table 3 shows M2/M1 (molar ratio) and the average molar absorption coefficient in the wavelength range of 200 nm to 380 nm of the M2 doped zinc oxide particles obtained in Example 1-1 to Example 1-15 together with the average molar absorption coefficient in the wavelength range of 200 nm to 380 nm of the zinc oxide particles obtained in Comparative Example 1. Since the average primary particle diameters are equal, it may be considered that the specific surface areas are equal. Table 3 also describes the increase rates of the average molar absorption coefficients (average molar absorption coefficient increase rate) of the M2 doped zinc oxide particles obtained in examples in the wavelength range of 200 nm to 380 nm, relative to the average molar absorption coefficient in the same wavelength range of the zinc oxide particles of Comparative Example 1. The concentrations of the elements of the above M2 other than Co in the dispersion at the time of molar absorption coefficient conversion, were converted to the molar absorption coefficient as $SiO_2$ for Si, $Fe_2O_3$ for Fe, $MnO_2$ for Mn, MgO for Mg and $Al_2O_3$ for Al.

Also, Table 3 shows L*, a* and b* measurement values, and hue H and chroma C calculated from the measurement values regarding the M2 doped zinc oxide particles obtained in Examples 1-1 to 1-19 and the zinc oxide particles obtained in Comparative Example 1.

[Table 3]

EP 3 467 071 B1

| Example | 1-1 | 1-2 | 1-3 | 1-4 | 1-5 | 1-6 | 1-7 | 1-8 | 1-9 | 1-10 | 1-11 | 1-12 | 1-13 | 1-14 | 1-15 | 1-16 | 1-17 | 1-18 | 1-19 | Comparative Example 1 |
|---|---|---|---|---|---|---|---|---|---|---|---|---|---|---|---|---|---|---|---|---|
| M2 | Co | Co | Co | Co | Co | Co+Si | Co+Si | Fe | Mn | Mg | Co+Al | Fe | Co+Al | Co+Al | Co | Fe | Mn | Co | Co | - |
| M1 | Zn | Zn | Zn | Zn | Zn | Zn | Zn | Zn | Zn | Zn | Zn | Zn | Zn | Zn | Zn | Zn | Zn | Zn | Zn | Zn |
| Molar ratio (M2/M1) | 0.015 | 0.053 | 0.111 | 0.333 | 1.000 | 0.178 | 0.401 | 0.015 | 0.015 | 0.015 | 1.000 | 0.042 | 1.000 | 1.000 | 0.111 | 0.015 | 0.015 | 0.015 | 0.015 | 0.000 |
| Average molar absorption coefficient [L/(mol·cm)] (200 to 380 nm) | 1103 | 955 | 900 | 858 | 869 | 1234 | 1331 | 1531 | 1364 | 1139 | 989 | 1641 | 979 | 946 | 2314 | 1796 | 1659 | 1239 | 1431 | 623 |
| Average molar absorption coefficient increase rate [%] | 177 | 153 | 144 | 138 | 139 | 198 | 214 | 246 | 219 | 183 | 159 | 263 | 157 | 152 | 371 | 288 | 266 | 199 | 230 | 100 |
| L* | 72.54 | 64.68 | 56.27 | 47.12 | 50.92 | 52.47 | 57.52 | 90.08 | 71.01 | 94.36 | 79.61 | 78.22 | 66.4 | 68.9 | 58.47 | 89.32 | 61.55 | 65.17 | 61.8 | 95.93 |
| a* | -16.33 | -25.4 | -18.7 | -6.38 | -7.38 | -5.62 | -4.86 | 3.39 | 26.71 | -2.36 | 2.03 | 8.92 | 6.81 | 21.23 | -16.48 | -8.61 | 17.53 | -24.69 | -13.58 | -0.41 |
| b* | -7.48 | -16.42 | -4.79 | -5.46 | -1.37 | -26.8 | -9.81 | 18.23 | 10.34 | 2.13 | -16.11 | 25.34 | -20.94 | -19.79 | -16.31 | 26.34 | 4.83 | 4.31 | 13.1 | 1.56 |
| Hue H | 0.46 | 0.65 | 0.26 | 0.86 | 0.19 | 4.77 | 2.02 | 5.38 | 0.39 | -0.90 | -7.94 | 2.84 | -3.07 | -0.93 | 0.99 | -3.06 | 0.28 | -0.17 | -0.96 | -3.80 |
| Chroma C | 17.96 | 30.25 | 19.30 | 8.40 | 7.51 | 27.38 | 10.95 | 18.54 | 28.64 | 3.18 | 16.24 | 26.86 | 22.02 | 29.02 | 23.19 | 27.71 | 18.18 | 25.06 | 18.87 | 1.61 |
| L*a*b* color system chromaticity diagram | ① | ② | ③ | ④ | ⑤ | ⑥ | ⑦ | ⑧ | ⑨ | ⑩ | ⑪ | ⑫ | ⑬ | ⑭ | ⑮ | ⑯ | ⑰ | ⑱ | ⑲ | ⑳ |

As seen from Table 3, it is understood that the average molar absorption coefficients in the wavelength range of 200 nm to 380 nm were improved as compared with one of the zinc oxide particles not doped with M2. It is understood that increase rates of average molar absorption coefficients in the wavelength range of 200 nm to 380 nm of dispersions in which the above M2 doped zinc oxide particles were dispersed in a dispersion medium, were improved relative to the average molar absorption coefficient in the same wavelength range of a dispersion of the zinc oxide particles not doped with M2. In addition, it is understood that in the case where at least a part of the surface of the particles was coated with a silicon compound (Example 1-15), the average molar absorption coefficient in the wavelength range of 200 nm to 380 nm was improved compared with the same oxide particles in which the surface of the particles was not coated with a silicon compound (Example 1-3). In any case, when used to a composition for coating or a composition for a transparent material, there is an advantage that a substance degraded by ultraviolet rays contained in a coating material or a transparent material can be protected, and degradation or decomposition and the like of an object by ultraviolet rays that have passed through a coating material or a transparent material can be efficiently protected. For example, in the case of a coated product, the above object is a skin in a human body, a base of a coated body, etc., and an indoor equipment or decorative product having a glass as a transparent material, or the like.

FIG 5 shows a chart of an L*a*b* color system chromaticity diagram, plotting L*, a*, b* shown in Table 3. As seen in FIG 5, it was found that the color characteristics can be strictly controlled by changing the species and concentration of M2, in the range of $40 \leq L^* \leq 95$, $-35 \leq a^* \leq 35$ or $-35 \leq b^* \leq 35$, preferably in the range of $40 \leq L^* \leq 95$, $-30 \leq a^* \leq 30$ or $-30 \leq b^* \leq 30$ in the L*a*b* color system. It was also found that the color characteristics can be strictly controlled in the above range by heat treatment performed as modification of a functional group or modification of oxidation number.

As described above, it was confirmed that ultraviolet shielding ability was improved by doping M2 to zinc oxide particles, and that various compositions having controlled color characteristics can be provided by controlling a molar ratio (M2/M1).

(Example 2)

As M2 doped oxide particles of Example 2, M2 doped iron oxide particles using iron as M1 were prepared. The particles were prepared in the same manner as in Example 1 except for matters shown in Table 4 and Table 5. The M2 doped iron oxide particles obtained in Example 2-3 were subjected with heat treatment using an electric furnace to change color characteristics. The powders of Example 2-3 were heated at 150 °C (Example 2-9), 200 °C (Example 2-10) and 300 °C (Example 2-11). The every heat treatment time was 30 minutes. Further, in the same manner as in Comparative Example 1, iron oxide particles not doped with M2 were prepared (Comparative Example 2). The analysis results obtained by the same method as in Example 1 are shown in Table 6. As for STEM and XRD measurement results, the similar results as in Example 1 were obtained. Regarding the substances represented by the chemical formula and abbreviations set forth in Table 4, $Fe(NO_3)_3 \cdot 9H_2O$ is iron nitrate nonahydrate (Kanto Kagaku Co., Ltd.), $Al(NO_3)_3 \cdot 9H_2O$ is aluminum nitrate nonahydrate (Kanto Kagaku Co., Ltd.), $Mg(NO_3)_2 \cdot 6H_2O$ is magnesium nitrate hexahydrate (Kanto Kagaku Co., Ltd.), $Mn(NO_3)_2 \cdot 6H_2O$ is manganese nitrate hexahydrate (Kanto Kagaku Co., Ltd.), 24wt% $Ti(SO_4)_2$ is titanium (IV) sulfate solution (Kanto Kagaku Co., Ltd., as >24.0%: $Ti(SO_4)_2$), $Zn(NO_3)_2 \cdot 6H_2O$ is zinc nitrate hexahydrate (Kanto Kagaku Co., Ltd.), TEOS is tetraethyl orthosilicate (Wako Pure Chemical Industry Ltd.), EG is ethylene glycol (Kishida Chemical Co., Ltd.), MeOH is methanol (Godo Co., Ltd.), and NaOH is sodium hydroxide (Kanto Chemical Co., Inc.). When converting the molar absorption coefficient of the M2 doped iron oxide particles obtained in Example 2-1 to Example 2-11 and the iron oxide particles obtained in Comparative Example 2, the absorption spectrum measurement results were converted to the molar absorption coefficient as $Fe_2O_3$ for Fe, CoO for Co, $MnO_2$ for Mn, $TiO_2$ for Ti, MgO for Mg and $Al_2O_3$ for Al.

[Table 4]

| | Formulation of the 1st fluid (liquid A) Oxide raw material liquid | | | | | Formulation of the 2nd fluid (liquid B) Oxide precipitation solvent | | | | Formulation of the 3rd fluid (liquid C) | | | | |
| | Formulation | | | pH | | Formulation | | pH | | Formulation | | | pH | |
| | Raw material [wt%] | Raw material [wt%] | Raw material [wt%] | pH | [°C] | Raw material [wt%] | Raw material [wt%] | pH | [°C] | Raw material [wt%] | Raw material [wt%] | Raw material [wt%] | pH | [°C] |
|---|---|---|---|---|---|---|---|---|---|---|---|---|---|---|
| Example 2-1 | Fe(NO₃)₃·9H₂O [2.000 wt%] | Al(NO₃)₃·9H₂O [0.028 wt%] | Pure water [97.972 wt%] | 1.81 | 23.0 | NaOH [9.0 wt%] | Pure water [91.0 wt%] | >14 | - | - | - | - | - | - |
| 2-2 | Fe(NO₃)₃·9H₂O [2.000 wt%] | Al(NO₃)₃·9H₂O [0.028 wt%] | Pure water [97.972 wt%] | 1.81 | 23.0 | NaOH [9.0 wt%] | Pure water [91.0 wt%] | >14 | - | - | - | - | - | - |
| 2-3 | Fe(NO₃)₃·9H₂O [2.000 wt%] | Al(NO₃)₃·9H₂O [0.028 wt%] | Pure water [97.972 wt%] | 1.81 | 23.0 | NaOH [9.0 wt%] | Pure water [91.0 wt%] | >14 | - | - | - | - | - | - |
| 2-4 | Fe(NO₃)₃·9H₂O [2.000 wt%] | Mg(NO₃)₂·6H₂O [0.039 wt%] | Pure water [97.961 wt%] | 1.19 | 21.4 | NaOH [9.0 wt%] | Pure water [91.0 wt%] | >14 | - | - | - | - | - | - |
| 2-5 | Fe(NO₃)₃·9H₂O [2.000 wt%] | Mn(NO₃)₂·6H₂O [0.044 wt%] | Pure water [97.956 wt%] | 1.81 | 24.9 | NaOH [9.0 wt%] | Pure water [91.0 wt%] | >14 | - | - | - | - | - | - |
| 2-6 | Fe(NO₃)₃·9H₂O [2.000 wt%] | 24wt% Ti(SO₄)₂ [0.153 wt%] | Pure water [97.847 wt%] | 1.45 | 26.5 | NaOH [9.0 wt%] | Pure water [91.0 wt%] | >14 | - | - | - | - | - | - |
| 2-7 | Fe(NO₃)₃·9H₂O [2.000 wt%] | Zn(NO₃)₂·6H₂O [1.360 wt%] | Pure water [96.640 wt%] | 1.45 | 26.5 | NaOH [9.0 wt%] | Pure water [91.0 wt%] | >14 | - | TEOS [0.57 wt%] | MeOH [2.43 wt%] | EG [97.00 wt%] | 6.13 | 16.1 |
| 2-8 | Fe(NO₃)₃·9H₂O [2.000 wt%] | Al(NO₃)₃·9H₂O [0.028 wt%] | Pure water [97.972 wt%] | 1.81 | 23.0 | NaOH [9.0 wt%] | Pure water [91.0 wt%] | >14 | - | - | - | - | - | - |
| Comparative Example 2 | Fe(NO₃)₃·9H₂O [2.000 wt%] | - [0.000 wt%] | Pure water [98.000 wt%] | 1.64 | 28.4 | NaOH [9.0 wt%] | Pure water [91.0 wt%] | >14 | - | - | - | - | - | - |

[Table 5]

| | Introduction flow rate (liquid sending flow rate) [ml/min] | | | Introduction temperature (liquid sending temperature) [°C] | | | Introduction pressure (liquid sending pressure) [MPaG] | | | Discharged liquid | | Molar ratio (M2/M1) | | | | Average primary particle diameter [nm] |
| | Liquid A | Liquid B | Liquid C | Liquid A | Liquid B | Liquid C | Liquid A | Liquid B | Liquid C | pH | Temperature [°C] | M2 | M1 | Metal oxide doped iron oxide particles [Calcurated value] | [EDS] | |
|---|---|---|---|---|---|---|---|---|---|---|---|---|---|---|---|---|
| Example 2-1 | 400 | 31 | - | 137 | 89 | - | 0.268 | 0.30 | - | 11.39 | 23.0 | Al | Fe | 0.015 | 0.015 | 9.66 |
| Example 2-2 | 400 | 30 | - | 137 | 86 | - | 0.255 | 0.30 | - | 5.69 | 23.3 | Al | Fe | 0.015 | 0.015 | 9.43 |
| Example 2-3 | 400 | 30.5 | - | 138 | 90 | - | 0.249 | 0.30 | - | 6.50 | 23.3 | Al | Fe | 0.015 | 0.015 | 9.79 |
| Example 2-4 | 400 | 40 | - | 137 | 86 | - | 0.276 | 0.30 | - | 12.64 | 30.5 | Mg | Fe | 0.031 | 0.031 | 9.68 |
| Example 2-5 | 400 | 40 | - | 137 | 89 | - | 0.278 | 0.30 | - | 12.62 | 26.1 | Mn | Fe | 0.031 | 0.031 | 9.98 |
| Example 2-6 | 400 | 40 | - | 138 | 83 | - | 0.283 | 0.30 | - | 12.64 | 19.7 | Ti | Fe | 0.031 | 0.031 | 9.93 |
| Example 2-7 | 400 | 38 | - | 138 | 83 | - | 0.283 | 0.30 | - | 9.86 | 21.6 | Zn | Fe | 0.923 | 0.031 | 9.93 |
| Example 2-8 | 400 | 31 | 100 | 137 | 89 | 88 | 0.268 | 0.30 | 0.30 | 11.06 | 24.3 | Al | Fe | 0.015 | 0.015 | 9.93 |
| Comparative Example 2 | 400 | 38 | - | 140 | 90 | - | 0.297 | 0.10 | - | 12.49 | 28.1 | - | - | - | - | 9.53 |

[Table 6]

| Example | 2-1 | 2-2 | 2-3 | 2-4 | 2-5 | 2-6 | 2-7 | 2-8 | 2-9 | 2-10 | 2-11 | Comparative Example 2 |
|---|---|---|---|---|---|---|---|---|---|---|---|---|
| M2 | Al | Al | Al | Mg | Mn | Ti | Zn | Al | Al | Al | Al | - |
| M1 | Fe | Fe | Fe | Fe | Fe | Fe | Fe | Fe | Fe | Fe | Fe | Fe |
| Molar ratio (M2/M1) | 0.015 | 0.015 | 0.015 | 0.031 | 0.031 | 0.031 | 0.923 | 0.015 | 0.015 | 0.015 | 0.015 | 0.000 |
| Average molar absorption coefficient [L/(mol·cm)] (200 to 380 nm) | 1536 | 1631 | 1769 | 2213 | 2439 | 2897 | 1697 | 1897 | 1898 | 2016 | 1946 | 765 |
| Average molar absorption coefficient increase rate [%] | 201 | 213 | 231 | 289 | 319 | 379 | 222 | 248 | 248 | 264 | 254 | 100 |
| L* | 41.04 | 43.08 | 40.00 | 40.9 | 39.4 | 41.34 | 78.6 | 40.32 | 38.2 | 38.45 | 40.04 | 43.81 |
| a* | 9.76 | 11.79 | 8.47 | 11.5 | 6.87 | 9.37 | 4.43 | 13.12 | 6.56 | 7.9 | 12.46 | 13.95 |
| b* | 8.91 | 11.52 | 7.68 | 9.28 | 6.27 | 7.88 | 6.54 | 6.27 | 4.67 | 5.13 | 7.15 | 13.44 |
| Hue H | 0.91 | 0.98 | 0.91 | 0.81 | 0.91 | 0.84 | 1.48 | 0.48 | 0.71 | 0.65 | 0.57 | 0.96 |
| Chroma C | 13.22 | 16.48 | 11.43 | 14.78 | 9.30 | 12.24 | 7.90 | 14.54 | 8.05 | 9.42 | 14.37 | 19.37 |
| L*a*b* color system chromaticity diagram | ① | ② | ③ | ④ | ⑤ | ⑥ | ⑦ | ⑧ | ⑨ | ⑩ | ⑪ | ⑫ |

As seen from Table 6, it is understood that the avarage molar absorption coefficients in the wavelenght range og 200 nm to 380 nm were improved as compared with one of the iron oxide particles not doped eith M2. It is understood thet increase rates of average molar absorption coefficients in the wavelenght range og 200 nm to 380 nm of dispersion in which the above M2 doped iron oxide perticles were dispersed in a dispersion medium, were improved relative to the average molar absorption coefficient in the same wavelength range of a dispersion of the iron oxide particles not doped with M2. In addition, it is understood that in the case where at least a part of the surface of the particles was coated with a silicon compound (Example 2-11), the average molar absorption coefficient in the wavelength range of 200 nm to 380 nm was improved compared with the same oxide particles in which the surface of the particles was not coated with a silicon compound (Example 2-1). As in the case of Example 1, in any case, when used to a composition for coating or a composition for a transparent material, there is an advantage that a substance degraded by ultraviolet rays contained in a coating material or a transparent material can be protected, and degradation or decomposition and the like of an object by ultraviolet rays that have passed through a coating material or a transparent material can be efficiently protected.

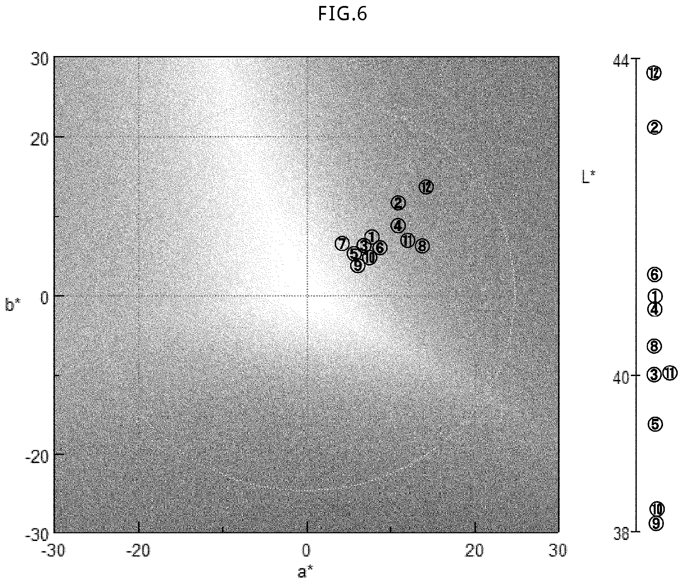

FIG 6 shows a chart of an L*a*b* color system chromaticity diagram, plotting L*, a*, b* shown in Table 6. L* of the M2 doped iron oxide particles obtained in Example 2-7 was largely different from those of the other M2 doped iron oxide particles, so that it was not plotted. As seen in FIG 6, it was found that the color characteristics can be strictly controlled by changing the species and concentration of M2, in the range of $38 \leq L^* \leq 44$, $4 \leq a^* \leq 14$ or $4 \leq b^* \leq 12$ in the L*a*b* color system. It was also found that the color characteristics can be strictly controlled in the above range by heat treatment performed as modification of a functional group or modification of oxidation number. Further, as seen from Example 2-1 to Example 2-3, even when M2/M1 was the same, L* value, a* value, b* value, hue or chroma can be changed, by changing the pH at precipitating M2 doped oxide particles.

As described above, similarly to Example 1, it was confirmed that ultraviolet shielding ability was improved by doping M2 to iron oxide particles, and that various compositions having controlled color characteristics can be provided by controlling a molar ratio (M2/M1).

(Example 3)

As M2 doped oxide particles of Example 3, M2 doped iron oxide particles using titanium as M1 were prepared. The particles were prepared in the same manner as in Example 1 except for matters shown in Table 7 and Table 8. Further, in the same manner as in Comparative Example 1, titanium oxide particles not doped with M2 were prepared (Comparative Example 3). The analysis results obtained by the same method as in Example 1 are shown in Table 9. As for STEM and XRD measurement results, the similar results as in Example 1 were obtained. Regarding the substances represented by the chemical formula and abbreviations set forth in Table 7, $TiOSO_4 \cdot nH_2O$ is titanyl sulfate (Kishida Chemical Co., Ltd.), TEOS is tetraethyl orthosilicate (Wako Pure Chemical Industry Ltd.), $Fe(NO_3)_3 \cdot 9H_2O$ is iron nitrate nonahydrate (Kanto Kagaku Co., Ltd.), $Co(NO_3)_2 \cdot 6H_2O$ is cobalt nitrate hexahydrate (Kanto Kagaku Co., Ltd.), $Mn(NO_3)_2 \cdot 6H_2O$ is manganese nitrate hexahydrate (Kanto Kagaku Co., Ltd.), 97% $H_2SO_4$ is concentrated sulfuric acid (Kishida Chemical Co., Ltd.), EG is ethylene glycol (Kishida Chemical Co., Ltd.), MeOH is methanol (Godo Co., Ltd.), and NaOH is sodium hydroxide (Kanto Chemical Co., Inc.). When converting the molar absorption coefficient of the M2 doped titanium oxide particles obtained in Example 3-1 to Example 3-17 and the titanium oxide particles obtained in Comparative Example 3, the absorption spectrum measurement results were converted to the molar absorption coefficient as $TiO_2$ for Ti, $Fe_2O_3$ for Fe, CoO for Co, $MnO_2$ for Mn and $SiO_2$ for Si.

[Table 7]

| | | Formulation of the 1st fluid (liquid A) Oxide raw material liquid | | | | | | Formulation of the 2nd fluid (liquid B) Oxide precipitation solvent | | | | Formulation of the 3rd fluid (liquid C) | | | | |
|---|---|---|---|---|---|---|---|---|---|---|---|---|---|---|---|---|
| | | Formulation | | | | pH | | Formulation | | pH | | Formulation | | | pH | |
| | | Raw material [wt%] | Raw material [wt%] | Raw material [wt%] | Raw material [wt%] | pH | [°C] | Raw material [wt%] | Raw material [wt%] | pH | [°C] | Raw material [wt%] | Raw material [wt%] | Raw material [wt%] | pH | [°C] |
| Example | 3-1 | $TiOSO_4 \cdot nH_2O$ [1.120 wt%] | TEOS [0.066 wt%] | 97wt% $H_2SO_4$ [1.000 wt%] | Pure water [97.814 wt%] | 1.05 | 13.6 | NaOH [9.00 wt%] | Pure water [91.00 wt%] | >14 | - | - | - | - | - | - |
| | 3-2 | $TiOSO_4 \cdot nH_2O$ [1.120 wt%] | TEOS [0.019 wt%] | 97wt% $H_2SO_4$ [1.000 wt%] | Pure water [97.861 wt%] | 1.16 | 14.1 | NaOH [9.00 wt%] | Pure water [91.00 wt%] | >14 | - | - | - | - | - | - |
| | 3-3 | $TiOSO_4 \cdot nH_2O$ [1.120 wt%] | $Fe(NO_3)_3 \cdot 9H_2O$ [0.081 wt%] | 97wt% $H_2SO_4$ [1.000 wt%] | Pure water [97.799 wt%] | 0.83 | 14.1 | NaOH [9.00 wt%] | Pure water [91.00 wt%] | >14 | - | - | - | - | - | - |
| | 3-4 | $TiOSO_4 \cdot nH_2O$ [1.120 wt%] | $Fe(NO_3)_3 \cdot 9H_2O$ [0.421 wt%] | 97wt% $H_2SO_4$ [1.000 wt%] | Pure water [97.459 wt%] | 0.78 | 14.2 | NaOH [9.00 wt%] | Pure water [91.00 wt%] | >14 | - | - | - | - | - | - |
| | 3-5 | $TiOSO_4 \cdot nH_2O$ [1.120 wt%] | $Fe(NO_3)_3 \cdot 9H_2O$ [0.919 wt%] | 97wt% $H_2SO_4$ [1.000 wt%] | Pure water [96.961 wt%] | 0.78 | 14.2 | NaOH [9.00 wt%] | Pure water [91.00 wt%] | >14 | - | - | - | - | - | - |
| | 3-6 | $TiOSO_4 \cdot nH_2O$ [1.120 wt%] | $Co(NO_3)_2 \cdot 6H_2O$ [0.304 wt%] | 97wt% $H_2SO_4$ [1.000 wt%] | Pure water [97.576 wt%] | 0.79 | 14.3 | NaOH [9.00 wt%] | Pure water [91.00 wt%] | >14 | - | - | - | - | - | - |
| | 3-7 | $TiOSO_4 \cdot nH_2O$ [1.120 wt%] | $Co(NO_3)_2 \cdot 6H_2O$ [0.048 wt%] | 97wt% $H_2SO_4$ [1.000 wt%] | Pure water [97.832 wt%] | 0.79 | 14.5 | NaOH [9.00 wt%] | Pure water [91.00 wt%] | >14 | - | - | - | - | - | - |
| | 3-8 | $TiOSO_4 \cdot nH_2O$ [1.120 wt%] | $Co(NO_3)_2 \cdot 6H_2O$ [0.332 wt%] | 97wt% $H_2SO_4$ [1.000 wt%] | Pure water [97.548 wt%] | 0.81 | 14.7 | NaOH [9.00 wt%] | Pure water [91.00 wt%] | >14 | - | - | - | - | - | - |
| | 3-9 | $TiOSO_4 \cdot nH_2O$ [1.120 wt%] | $Co(NO_3)_2 \cdot 6H_2O$ [0.788 wt%] | 97wt% $H_2SO_4$ [1.000 wt%] | Pure water [97.092 wt%] | 0.83 | 14.9 | NaOH [9.00 wt%] | Pure water [91.00 wt%] | >14 | - | - | - | - | - | - |
| | 3-10 | $TiOSO_4 \cdot nH_2O$ [1.120 wt%] | $Mn(NO_3)_2 \cdot 6H_2O$ [0.299 wt%] | 97wt% $H_2SO_4$ [1.000 wt%] | Pure water [97.581 wt%] | 0.76 | 14.6 | NaOH [9.00 wt%] | Pure water [91.00 wt%] | >14 | - | - | - | - | - | - |
| | 3-11 | $TiOSO_4 \cdot nH_2O$ [1.120 wt%] | $Mn(NO_3)_2 \cdot 6H_2O$ [0.577 wt%] | 97wt% $H_2SO_4$ [1.000 wt%] | Pure water [97.303 wt%] | 0.73 | 15.1 | NaOH [9.00 wt%] | Pure water [91.00 wt%] | >14 | - | - | - | - | - | - |
| | 3-12 | $TiOSO_4 \cdot nH_2O$ [1.120 wt%] | $Mn(NO_3)_2 \cdot 6H_2O$ [0.777 wt%] | 97wt% $H_2SO_4$ [1.000 wt%] | Pure water [97.103 wt%] | 0.72 | 14.9 | NaOH [9.00 wt%] | Pure water [91.00 wt%] | >14 | - | - | - | - | - | - |
| | 3-13 | $TiOSO_4 \cdot nH_2O$ [1.120 wt%] | $Mn(NO_3)_2 \cdot 6H_2O$ [0.048 wt%] | 97wt% $H_2SO_4$ [1.000 wt%] | Pure water [97.832 wt%] | 0.81 | 14.5 | NaOH [9.00 wt%] | Pure water [91.00 wt%] | >14 | - | - | - | - | - | - |
| | 3-14 | $TiOSO_4 \cdot nH_2O$ [1.120 wt%] | TEOS [0.019 wt%] | 97wt% $H_2SO_4$ [1.000 wt%] | Pure water [97.861 wt%] | 1.16 | 14.1 | NaOH [9.00 wt%] | Pure water [91.00 wt%] | >14 | - | TEOS [0.57 wt%] | MeOH [2.43 wt%] | EG [97.00 wt%] | 6.13 | 16.1 |
| | 3-15 | $TiOSO_4 \cdot nH_2O$ [1.120 wt%] | TEOS [0.154 wt%] | 97wt% $H_2SO_4$ [1.000 wt%] | Pure water [97.726 wt%] | 1.18 | 14.1 | NaOH [9.00 wt%] | Pure water [91.00 wt%] | >14 | - | TEOS [0.57 wt%] | MeOH [2.43 wt%] | EG [97.00 wt%] | 6.13 | 16.1 |
| | 3-16 | $TiOSO_4 \cdot nH_2O$ [1.120 wt%] | TEOS [0.423 wt%] | 97wt% $H_2SO_4$ [1.000 wt%] | Pure water [97.457 wt%] | 1.21 | 14.3 | NaOH [9.00 wt%] | Pure water [91.00 wt%] | >14 | - | TEOS [0.57 wt%] | MeOH [2.43 wt%] | EG [97.00 wt%] | 6.13 | 16.1 |
| | 3-17 | $TiOSO_4 \cdot nH_2O$ [1.120 wt%] | TEOS [0.552 wt%] | 97wt% $H_2SO_4$ [1.000 wt%] | Pure water [97.328 wt%] | 1.23 | 14.1 | NaOH [9.00 wt%] | Pure water [91.00 wt%] | >14 | - | TEOS [0.57 wt%] | MeOH [2.43 wt%] | EG [97.00 wt%] | 6.13 | 16.1 |
| Comparative Example 3 | | $TiOSO_4 \cdot nH_2O$ [1.120 wt%] | - [0.000 wt%] | 97wt% $H_2SO_4$ [1.000 wt%] | Pure water [97.880 wt%] | 1.08 | 20.6 | NaOH [9.00 wt%] | Pure water [91.00 wt%] | >14 | - | - | - | - | - | - |

26

[Table 8]

| | | Introduction flow rate (liquid sending flow rate) | | | Introduction temperature (liquid sending temperature) | | | Introduction pressure (liquid sending pressure) | | | Discharged liquid | | Molar ratio (M2/M1) | | | | Average primary particle diameter |
|---|---|---|---|---|---|---|---|---|---|---|---|---|---|---|---|---|---|
| | | [ml/min] | | | [°C] | | | [MPaG] | | | pH | Temperature [°C] | M2 | M1 | Metal oxide doped titanium oxide particles | | [nm] |
| | | Liquid A | Liquid B | Liquid C | Liquid A | Liquid B | Liquid C | Liquid A | Liquid B | Liquid C | | | | | [Calcurated value] | [EDS] | |
| Example | 3-1 | 200 | 50 | - | 133 | 78 | - | 0.297 | 0.30 | - | 13.08 | 17.0 | Si | Ti | 0.101 | 0.101 | 11.23 |
| | 3-2 | 200 | 50 | - | 137 | 86 | - | 0.277 | 0.30 | - | 11.93 | 43.2 | Si | Ti | 0.029 | 0.029 | 11.36 |
| | 3-3 | 200 | 50 | - | 136 | 90 | - | 0.234 | 0.30 | - | 9.94 | 39.3 | Fe | Ti | 0.064 | 0.064 | 11.39 |
| | 3-4 | 100 | 50 | - | 135 | 88 | - | 0.269 | 0.30 | - | 10.39 | 29.1 | Fe | Ti | 0.333 | 0.333 | 11.82 |
| | 3-5 | 100 | 50 | - | 135 | 88 | - | 0.59 | 0.30 | - | 10.21 | 28.4 | Fe | Ti | 0.726 | 0.726 | 11.92 |
| | 3-6 | 400 | 50 | - | 136 | 89 | - | 0.298 | 0.30 | - | 9.59 | 28.7 | Co | Ti | 0.333 | 0.333 | 11.49 |
| | 3-7 | 400 | 50 | - | 134 | 84 | - | 0.229 | 0.30 | - | 10.57 | 23.7 | Co | Ti | 0.053 | 0.053 | 11.72 |
| | 3-8 | 400 | 50 | - | 134 | 82 | - | 0.231 | 0.30 | - | 10.62 | 23.6 | Co | Ti | 0.364 | 0.364 | 11.61 |
| | 3-9 | 400 | 50 | - | 134 | 84 | - | 0.236 | 0.30 | - | 10.59 | 23.9 | Co | Ti | 0.864 | 0.864 | 11.31 |
| | 3-10 | 400 | 50 | - | 138 | 90 | - | 0.297 | 0.30 | - | 10.10 | 28.8 | Mn | Ti | 0.333 | 0.333 | 11.24 |
| | 3-11 | 400 | 50 | - | 138 | 90 | - | 0.289 | 0.30 | - | 10.09 | 29.1 | Mn | Ti | 0.642 | 0.642 | 11.31 |
| | 3-12 | 400 | 50 | - | 138 | 90 | - | 0.292 | 0.30 | - | 10.12 | 28.9 | Mn | Ti | 0.864 | 0.864 | 11.19 |
| | 3-13 | 100 | 50 | - | 139 | 90 | - | 0.299 | 0.30 | - | 10.67 | 20.5 | Mn | Ti | 0.053 | 0.053 | 11.39 |
| | 3-14 | 200 | 50 | 50 | 133 | 78 | 89 | 0.297 | 0.30 | 0.30 | 11.67 | 18.6 | Si | Ti | 0.029 | 0.029 | 11.49 |
| | 3-15 | 200 | 50 | 50 | 133 | 78 | 89 | 0.297 | 0.30 | 0.30 | 11.90 | 18.9 | Si | Ti | 0.236 | 0.236 | 11.51 |
| | 3-16 | 200 | 50 | 50 | 133 | 78 | 89 | 0.297 | 0.30 | 0.30 | 11.87 | 18.9 | Si | Ti | 0.648 | 0.648 | 11.48 |
| | 3-17 | 200 | 50 | 50 | 133 | 78 | 89 | 0.297 | 0.30 | 0.30 | 11.76 | 19.1 | Si | Ti | 0.846 | 0.846 | 11.64 |
| Comparative Example 3 | | 400 | 50 | - | 140 | 90 | - | 0.297 | 0.10 | - | 11.98 | 25.6 | - | - | - | - | 11.36 |

[Table 9]

| Example | 3-1 | 3-2 | 3-3 | 3-4 | 3-5 | 3-6 | 3-7 | 3-8 | 3-9 | 3-10 | 3-11 | 3-12 | 3-13 | 3-14 | 3-15 | 3-16 | 3-17 | Comparative Example 3 |
|---|---|---|---|---|---|---|---|---|---|---|---|---|---|---|---|---|---|---|
| M2 | Si | Si | Fe | Fe | Fe | Co | Co | Co | Co | Mn | Mn | Mn | Mn | Si | Si | Si | Si | - |
| M1 | Ti | Ti | Ti | Ti | Ti | Ti | Ti | Ti | Ti | Ti | Ti | Ti | Ti | Ti | Ti | Ti | Ti | Ti |
| Molar ratio (M2/M1) | 0.101 | 0.029 | 0.064 | 0.333 | 0.726 | 0.333 | 0.053 | 0.364 | 0.864 | 0.333 | 0.642 | 0.864 | 0.053 | 0.029 | 0.236 | 0.648 | 0.846 | 0.000 |
| Average molar absorption coefficient [L/(mol·cm)] (200 to 380 nm) | 6134 | 6239 | 6319 | 6449 | 6516 | 6239 | 6129 | 6326 | 6274 | 6646 | 6714 | 6802 | 6128 | 6697 | 6749 | 6801 | 6969 | 5168 |
| Average molar absorption coefficient increase rate [%] | 119 | 121 | 122 | 125 | 126 | 121 | 119 | 122 | 121 | 129 | 130 | 132 | 119 | 130 | 131 | 132 | 135 | 100 |
| L* | 94.87 | 93.32 | 93.24 | 84.40 | 76.30 | 68.55 | 78.34 | 54.31 | 41.10 | 59.45 | 51.10 | 43.60 | 79.62 | 94.16 | 87.40 | 73.20 | 57.40 | 95.78 |
| a* | -0.96 | -2.84 | -0.97 | 0.79 | 1.56 | -4.47 | -5.13 | -18.63 | -27.90 | 5.52 | 16.40 | 26.90 | 3.97 | -1.56 | 3.31 | -6.94 | -7.68 | -0.17 |
| b* | 1.01 | 3.86 | 7.66 | 16.21 | 27.20 | -3.40 | 0.66 | -2.86 | 5.52 | 6.38 | 6.30 | 7.90 | 2.55 | -6.81 | -11.10 | -18.60 | -27.10 | -1.02 |
| Hue H | -1.05 | -1.36 | -7.90 | 20.52 | 17.44 | 0.76 | -0.13 | 0.15 | -0.20 | 1.16 | 0.38 | 0.29 | 0.64 | 4.37 | -3.35 | 2.68 | 3.53 | 6.00 |
| Chroma C | 1.39 | 4.79 | 7.72 | 16.23 | 27.24 | 5.62 | 5.17 | 18.85 | 28.44 | 8.44 | 17.57 | 28.04 | 4.72 | 6.99 | 11.58 | 19.85 | 28.17 | 1.03 |
| L*a*b* color system chromaticity diagram | ① | ② | ③ | ④ | ⑤ | ⑥ | ⑦ | ⑧ | ⑨ | ⑩ | ⑪ | ⑫ | ⑬ | ⑭ | ⑮ | ⑯ | ⑰ | ⑱ |

As seen from Table 9, it is understood that the average molar absorption coefficients in the wavelength range of 200 nm to 380 nm were improved as compared with one of the titanium oxide particles not doped with M2. It is understood that increase rates of average molar absorption coefficients in the wavelength range of 200 nm to 380 nm of dispersions in which the above M2 doped titanium oxide particles were dispersed in a dispersion medium, were improved relative to the average molar absorption coefficient in the same wavelength range of a dispersion of the titanium oxide particles not doped with M2. In addition, it is understood that in the case where at least a part of the surface of the particles was coated with a silicon compound (Example 3-14), the average molar absorption coefficient in the wavelength range of 200 nm to 380 nm was improved compared with the same oxide particles in which the surface of the particles was not coated with a silicon compound (Example 3-2). As in the case of Example 1, in any case, when used to a composition for coating or a composition for a transparent material, there is an advantage that a substance degraded by ultraviolet rays contained in a coating material or a transparent material can be protected, and degradation or decomposition and the like of an object by ultraviolet rays that have passed through a coating material or a transparent material can be efficiently protected.

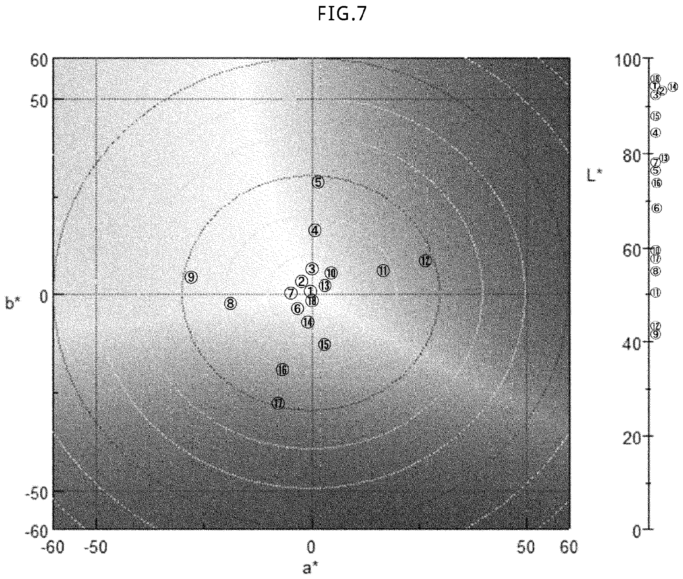

FIG 7 shows a chart of an L*a*b* color system chromaticity diagram, plotting L*, a*, b* shown in Table 9. As seen in FIG 7, it was found that the color characteristics can be strictly controlled by changing the species and concentration of M2, in the range of $40 \leq L^* \leq 95$, $-35 \leq a^* \leq 35$ or $-35 \leq b^* \leq 35$, preferably in the range of $40 \leq L^* \leq 95$, $-30 \leq a^* \leq 30$ or $-30 \leq b^* \leq 30$ in the L*a*b* color system.

As described above, similarly to Example 1, it was confirmed that ultraviolet shielding ability was improved by doping M2 to titanium oxide particles, and that various compositions having controlled color characteristics can be provided by controlling a molar ratio (M2/M1).

(Reference Example 4)

In Example 4, the M2 doped zinc oxide particles were prepared in the same manner as in Example 1 except for using an apparatus described in JP 2009-112892, and using a method of mixing and reacting liquid A (metal element doped zinc oxide raw material liquid), liquid B (oxide precipitation solvent) and liquid C (silicon oxide raw material liquid). Here, the apparatus of JP 2009-112892 was an apparatus described in FIG 1 of JP 2009-112892, and the inner diameter of the stirring tank was 80 mm, and the gap between the outer end of the mixing tool and the inner peripheral surface of the stirring tank was 0.5 mm, and the rotor rotational speed of the stirring blade was 7,200 rpm. Further, liquid A was introduced into the stirring tank, and liquid B was added, mixed and reacted in the thin film consisting of liquid A that was crimped to the inner peripheral surface of the stirring tank. As a result of TEM observation, the M2 doped zinc oxide particles having a primary particle diameter of about 20 nm to 30 nm were observed. Further, in the same manner as in Comparative Example 1, zinc oxide particles not doped with M2 were prepared (Comparative Example 4).

The results of the mapping and linear analysis using the STEM and the XRD measurement result of the M2 doped zinc oxide particles obtained in Example 4-1 to Example 4-8 showed similar results as in Example 1 (No drawings shown).

Table 10 shows the results of evaluating the M2 doped zinc oxide particles obtained in Example 4-1 to Example 4-8 in the same manner as in Example 1. When converting the molar absorption coefficient of the M2 doped zinc oxide particles obtained in Example 4-1 to Example 4-8 and the zinc oxide particles obtained in Comparative Example 4, the absorption spectrum measurement results were converted to the molar absorption coefficient as ZnO for Zn, $Fe_2O_3$ for Fe, CoO for Co, $MnO_2$ for Mn, $SiO_2$ for Si, MgO for Mg and $Al_2O_3$ for Al.

[Table 10]

| Example | 4-1 | 4-2 | 4-3 | 4-4 | 4-5 | 4-6 | 4-7 | 4-8 | Comparative Example 4 |
|---|---|---|---|---|---|---|---|---|---|
| M2 | Co | Co | Co | Co+Si | Fe | Mn | Mg | Co+Al | - |
| M1 | Zn | Zn | Zn | Zn | Zn | Zn | Zn | Zn | Zn |
| Molar ratio (M2/M1) | 0.015 | 0.111 | 1.000 | 0.401 | 0.015 | 0.015 | 0.015 | 1.000 | 0.000 |
| Average molar absorption coefficient [L/(mol·cm)] (200 to 380 nm) | 1103 | 900 | 869 | 1331 | 1531 | 1364 | 1139 | 989 | 623 |
| Average molar absorption coefficient increase rate [%] | 177 | 144 | 139 | 214 | 246 | 219 | 183 | 159 | 100 |
| L* | 72.48 | 56.23 | 50.68 | 57.31 | 89.91 | 70.63 | 93.99 | 79.54 | 95.79 |
| a* | -16.31 | -15.49 | -9.3 | -7.39 | -1.89 | 4.49 | -0.49 | -4.08 | -0.3 |

(continued)

| Example | 4-1 | 4-2 | 4-3 | 4-4 | 4-5 | 4-6 | 4-7 | 4-8 | Comparative Example 4 |
|---|---|---|---|---|---|---|---|---|---|
| b* | -5.72 | -6.44 | 1.29 | -7.88 | 13.96 | 16 | 2.29 | -8.49 | 1.34 |
| Hue H | 0.35 | 0.42 | -0.14 | 1.07 | -7.39 | 3.56 | -4.67 | 2.08 | -4.47 |
| Chroma C | 17.28 | 16.78 | 9.39 | 10.80 | 14.09 | 16.62 | 2.34 | 9.42 | 1.37 |

As seen from Table 10, even when M2 doped zinc oxide particles were prepared by using a device different from the device described in Patent Literature 6 or 7 unlike Example 1, it was confirmed that ultraviolet shielding ability was improved by doping another metallic element other than zinc to zinc oxide particles, and that various compositions having controlled color characteristics can be provided by controlling a molar ratio (M2/M1).

**Claims**

1. A coloring ultraviolet protective agent, which is useful for shielding ultraviolet rays and coloring, wherein:

   the coloring ultraviolet protective agent comprises M2 doped oxide particles in which oxide particles (M1Ox) comprising at least M1 being a metal element or metalloid element, are doped with at least one M2 selected from metal elements or metalloid elements other than M1,
   x is an arbitrary positive number,
   at least a part of the surface of the M2 doped oxide particles is coated with a compound containing an amorphous silicon oxide,
   a molar ratio of M1 and M2 (M2/M1) of the M2 doped oxide particles is in the range of 0.01 or more and 1.00 or less, and
   the M2 doped oxide particles are either one of the following:

   A) M1 is iron (Fe), color characteristics in the visible region of the M2 doped oxide particles are controlled in the range of $38 \leq L^* \leq 44$, $4 \leq a^* \leq 14$, or $4 \leq b^* \leq 12$ in the $L^*a^*b^*$ color system, and an average molar absorption coefficient in the wavelength range of 200 nm to 380 nm of a dispersion in which the M2 doped oxide particles are dispersed in a dispersion medium, is 1,000 L/(mol·cm) or more; or
   B) M1 is titanium (Ti), color characteristics in the visible region of the M2 doped oxide particles are controlled in the range of $40 \leq L^* \leq 95$, $-35 \leq a^* \leq 35$, or $-35 \leq b^* \leq 35$ in the $L^*a^*b^*$ color system, and an average molar absorption coefficient in the wavelength range of 200 nm to 380 nm of a dispersion in which the M2 doped oxide particles are dispersed in a dispersion medium, is 3,500 L/(mol·cm) or more.

2. The coloring ultraviolet protective agent according to claim 1,
   wherein an average molar absorption coefficient increase rate which is an increase rate of an average molar absorption coefficient in the wavelength range of 200 nm to 380 nm of a dispersion in which the M2 doped oxide particles are dispersed in a dispersion medium, over an average molar absorption coefficient in the same wavelength range of a dispersion in which the oxide particles (M1Ox) are dispersed in a dispersion medium is 110% or more.

3. The coloring ultraviolet protective agent according to claim 1 or claim 2, wherein an increase rate of the average molar absorption coefficient in the wavelength range of 200 nm to 380 nm of a dispersion in which the M2 doped oxide particles are dispersed in a dispersion medium, over an average molar absorption coefficient in the same wavelength range of a dispersion in which the oxide particles (M1Ox) are dispersed in a dispersion medium is controlled.

4. The coloring ultraviolet protective agent according to any one of claims 1 to 3, wherein the M2 doped oxide particles are solid solution oxide particles in which M1 and M2 are detected throughout the M2 doped oxide particles in STEM mapping.

5. The coloring ultraviolet protective agent according to any one of claims 1 to 4, wherein a primary particle diameter of the M2 doped oxide particle is 1 nm or more and 100 nm or less.

6. The coloring ultraviolet protective agent according to any one of claims 1 to 5, which is silicon compound coated M2 doped oxide particles in which at least a part of the surface of the M2 doped oxide particles is coated with a silicon compound,

wherein an average molar absorption coefficient in the wavelength range of 200 nm to 380 nm of a dispersion in which the silicon compound coated M2 doped oxide particles are dispersed in a dispersion medium, is increased over one of a dispersion of the M2 doped oxide particles not coated with the silicon compound.

**7.** A coloring ultraviolet protective agent composition, comprising the coloring ultraviolet protective agent according to any one of claims 1 to 6.

**Patentansprüche**

**1.** Färbendes UV-Schutzmittel, das zum Abschirmen von UV-Strahlen und zum Färben nützlich ist, wobei:

das färbende UV-Schutzmittel mit M2 dotierte Oxidteilchen umfasst, in welchen Oxidteilchen (M1Ox), die mindestens M1 umfassen, das ein Metallelement oder Metalloidelement ist, mit mindestens einem M2 dotiert sind, das aus Metallelementen oder Metalloidelementen ausgewählt ist, die von M1 verschieden sind, x eine willkürliche positive Zahl ist, mindestens ein Teil der Oberfläche der mit M2 dotierten Oxidteilchen mit einer Verbindung beschichtet ist, die amorphes Siliziumoxid enthält, ein Molverhältnis von M1 und M2 (M2/M1) der mit M2 dotierten Oxidteilchen im Bereich von 0,01 oder mehr und 1,00 oder weniger liegt, und die mit M2 dotierten Oxidteilchen eine der beiden folgenden Formen aufweisen:

A) M1 ist Eisen (Fe), die Farbeigenschaften im sichtbaren Bereich der mit M2 dotierten Oxidpartikel werden im Bereich von $38 \leq L^* \leq 44$, $4 \leq a^* \leq 14$, oder $4 \leq b^* \leq 12$ im L*a*b*-Farbsystem gesteuert, und ein durchschnittlicher molarer Absorptionskoeffizient im Wellenlängenbereich von 200 nm bis 380 nm einer Dispersion, in der die mit M2 dotierten Oxidpartikel in einem Dispersionsmedium dispergiert sind, beträgt 1 000 L/(Mol·cm) oder mehr; oder
B) M1 ist Eisen (Fe), die Farbeigenschaften im sichtbaren Bereich der mit M2 dotierten Oxidpartikel werden im Bereich von $40 \leq L^* \leq 95$, $-35 \leq a^* \leq 35$, oder $-35 \leq b^* \leq 35$ im L*a*b*-Farbsystem gesteuert, und ein durchschnittlicher molarer Absorptionskoeffizient im Wellenlängenbereich von 200 nm bis 380 nm einer Dispersion, in der die mit M2 dotierten Oxidpartikel in einem Dispersionsmedium dispergiert sind, beträgt 3 500 L/(Mol·cm) oder mehr; oder

**2.** Färbendes UV-Schutzmittel nach Anspruch 1, wobei eine Anstiegsrate des durchschnittlichen molaren Absorptionskoeffizienten, die eine Anstiegsrate eines durchschnittlichen molaren Absorptionskoeffizienten im Wellenlängenbereich von 200 nm bis 380 nm einer Dispersion ist, in der die mit M2 dotierten Oxidpartikel in einem Dispersionsmedium dispergiert sind, über einem durchschnittlichen molaren Absorptionskoeffizienten im gleichen Wellenlängenbereich einer Dispersion, in der die Oxidpartikel (M1Ox) in einem Dispersionsmedium dispergiert sind, 110 % oder mehr beträgt.

**3.** Färbendes UV-Schutzmittel nach Anspruch 1 oder 2, wobei eine Anstiegsrate des durchschnittlichen molaren Absorptionskoeffizienten im Wellenlängenbereich von 200 nm bis 380 nm einer Dispersion, in der die mit M2 dotierten Oxidpartikel in einem Dispersionsmedium dispergiert sind, über einem durchschnittlichen molaren Absorptionskoeffizienten im gleichen Wellenlängenbereich einer Dispersion, in der die Oxidpartikel (M1Ox) in einem Dispersionsmedium dispergiert sind, gesteuert wird.

**4.** Färbendes UV-Schutzmittel nach einem der Ansprüche 1 bis 3, wobei die mit M2 dotierten Oxidpartikel feste Lösungsoxidpartikel sind, wobei M1 und M2 überall in den mit M2 dotierten Oxidpartikeln in einer STEM-Kartierung nachgewiesen werden.

**5.** Färbendes UV-Schutzmittel nach einem der Ansprüche 1 bis 4, wobei ein Primärpartikeldurchmesser der mit M2 dotierten Oxidpartikel 1 nm oder mehr und 100 nm oder weniger beträgt.

**6.** Färbendes UV-Schutzmittel nach einem der Ansprüche 1 bis 5, das aus mit einer Siliziumverbindung beschichteten, mit M2 dotierten Oxidpartikeln besteht, wobei mindestens ein Teil der Oberfläche der mit M2 dotierten Oxidpartikel mit einer Siliziumverbindung beschichtet ist, wobei ein durchschnittlicher molarer Absorptionskoeffizient im Wellenlängenbereich von 200 nm bis 380 nm einer Dispersion, in der die mit einer Siliziumverbindung beschichteten, mit M2 dotierten Oxidpartikel in einem Dispersionsmedium dispergiert sind, im Vergleich zu einer Dispersion der mit M2 dotierten Oxidpartikel, die nicht mit der Siliziumverbindung beschichtet sind, erhöht ist.

7. Färbende UV-Schutzmittelzusammensetzung, umfassend das färbende UV-Schutzmittel nach einem der Ansprüche 1 bis 6.

**Revendications**

1. **Agent** colorant de protection contre les ultraviolets, qui est utile pour protéger contre les rayons ultraviolets et la coloration, dans lequel :

   l'agent colorant de protection contre les ultraviolets comprend des particules d'oxyde dopées par M2 dans lesquelles des particules d'oxyde (M1Ox), qui comprennent au moins M1 qui est un élément métallique ou un élément métalloïde, sont dopées avec au moins un M2 sélectionné parmi des éléments métalliques ou des éléments métalloïdes autres que M1,
   x est un nombre positif arbitraire,
   au moins une partie de la surface des particules d'oxyde dopées par M2 est revêtue d'un composé contenant un oxyde de silicium amorphe,
   un rapport molaire de M1 à M2 (M2/M1) des particules d'oxyde dopées par M2 se situe dans la plage allant de 0,01 ou plus et 1,00 ou moins, et
   les particules d'oxyde dopées par M2 sont l'une ou l'autre des suivantes :

      A) M1 est du fer (Fe), les caractéristiques de couleur dans la région visible des particules d'oxyde dopées par M2 sont régulées dans la plage de $38 \leq L^* \leq 44$, $4 \leq a^* \leq 14$ ou $4 \leq b^* \leq 12$ dans le système de couleurs L*a*b* et un coefficient d'absorption molaire moyen dans la plage de longueurs d'onde de 200 nm à 380 nm d'une dispersion dans laquelle les particules d'oxyde dopées par M2 sont dispersées dans un milieu de dispersion est de 1 000 L (mol·cm) ou plus ; ou
      B) M1 est du titane (Ti), les caractéristiques de couleur dans la région visible des particules d'oxyde dopées par M2 sont régulées dans la plage de $40 \leq L^* \leq 95$, $-35 \leq a^* \leq 35$ ou $-35 \leq b^* \leq 35$ dans le système de couleurs L*a*b* et un coefficient d'absorption molaire moyen dans la plage de longueurs d'onde de 200 nm à 380 nm d'une dispersion dans laquelle les particules d'oxyde dopées par M2 sont dispersées dans un milieu de dispersion est de 3 500 L (mol·cm) ou plus.

2. Agent colorant de protection contre les ultraviolets selon la revendication 1, dans lequel un taux d'augmentation de coefficient d'absorption molaire moyen qui est un taux d'augmentation d'un coefficient d'absorption molaire moyen dans la plage de longueurs d'onde de 200 nm à 380 nm d'une dispersion dans laquelle les particules d'oxyde dopées par M2 sont dispersées dans un milieu de dispersion sur un coefficient d'absorption molaire moyen dans la même plage de longueurs d'onde d'une dispersion dans laquelle les particules d'oxyde (M1Ox) sont dispersées dans un milieu de dispersion est de 110 % ou plus.

3. Agent colorant de protection contre les ultraviolets selon la revendication 1 ou la revendication 2, dans lequel un taux d'augmentation du coefficient d'absorption molaire moyen dans la plage de longueurs d'onde de 200 nm à 380 nm d'une dispersion dans laquelle les particules d'oxyde dopées par M2 sont dispersées dans un milieu de dispersion sur un coefficient d'absorption molaire moyen dans la même plage de longueurs d'onde d'une dispersion dans laquelle les particules d'oxyde (M1Ox) sont dispersées dans un milieu de dispersion est régulé.

4. Agent colorant de protection contre les ultraviolets selon l'une quelconque des revendications 1 à 3, dans lequel les particules d'oxyde dopées par M2 sont des particules d'oxyde en solution solides dans lesquelles M1 et M2 sont détectées dans l'ensemble des particules d'oxyde dopées par M2 dans un mappage STEM.

5. Agent colorant de protection contre les ultraviolets selon l'une quelconque des revendications 1 à 4, dans lequel un diamètre de particule primaire de la particule d'oxyde dopée par M2 est de 1 nm ou plus et de 100 nm ou moins.

6. Agent colorant de protection contre les ultraviolets selon l'une quelconque des revendications 1 à 5, qui est constitué de particules d'oxyde dopées par M2 revêtues de composé de silicium dans lesquelles au moins une partie de la surface des particules d'oxyde dopées par M2 est revêtue d'un composé de silicium,

dans lequel un coefficient d'absorption molaire moyen dans la plage de longueurs d'onde de 200 nm à 380 nm d'une dispersion, dans laquelle les particules d'oxyde dopées par M2 revêtues de composé de silicium sont dispersées dans un milieu de dispersion, est augmenté sur l'une d'une dispersion des particules d'oxyde dopées par M2 non revêtues du composé de silicium.

7. Composition d'agent colorant de protection contre les ultraviolets, comprenant l'agent colorant de protection contre les ultraviolets selon l'une quelconque des revendications 1 à 6.

## FIG.1

(a) HAADF

(b) O

(c) Zn

(d) Al

(e) Co

FIG.2

FIG.3

FIG.4

FIG.5

FIG.6

FIG.7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009263547 A
- WO 199826011 A
- JP 2013249393 A
- JP 2013520532 A
- JP 2001058821 A
- JP 4868558 B
- WO 2009008393 A
- JP 2005263612 A
- US 6534044 B1
- JP 2009112892 A
- JP 2014042891 A
- JP 2014042892 A